# EUROPEAN PATENT APPLICATION

(11) **EP 1 721 972 A2**
(43) Date of publication of application: **15.11.2006**
(21) Application number: 05719579.4
(22) Date of filing: 21.02.2005
(51) Int. Cl.: C12N 15/09, C07K 14/47, C07K 16/18, C12N 1/15, C12N 1/19, C12N 1/21, C12N 5/10, C12P 21/02, C12Q 1/68, G01N 33/15, G01N 33/50, G01N 33/53

(54) **TELOMERE PROTEIN TRF2 DNA-BINDING DOMAIN MUTANT PROTEIN, TELOMERE DNA MUTANT AND UTILIZATION OF COMPLEX STRUCTURE OF TRF2 DNA-BINDING DOMAIN WITH DOUBLE-STRANDED DNA**

(30) Priority: 23.02.2004 JP 2004046238
(71) Applicant: Yokohama City University, Yokohama-shi Kanagawa 236-0027 (JP)
(72) Inventor: NISHIMURA, Yoshifumi, 1810013 (JP); HANAOKA, Shingo, 3340058 (JP)
(74) Representative: Woods, Geoffrey Corlett
(86) International application number: PCT/JP2005/003237
(87) International publication number: WO 2005/086595

(57) **Abstract**

A TRF2 DNA-binding domain mutant protein comprising:
(a) a TRF2 DNA-binding domain mutant protein having an amino acid sequence as shown in SEQ ID NO: 2 but with at least one substitution selected from the group consisting of substitution of the lysine residue with arginine at position 10, substitution of the alanine residue with serine at position 34, substitution of the alanine residue with serine at position 47 and substitution of the arginine residue with lysine at position 59; or
(b) a TRF2 DNA-binding domain mutant protein having an amino acid sequence of the mutant protein of (a) above but with one or several amino acid residues other than the amino acid residues at positions 10, 34, 47 and 59 being deleted, substituted or added, and which has a higher binding ability to a duplex DNA comprising a sequence represented by 5'-TTAGGG-3' than a wild-type TRF2 DNA-binding domain protein having an amino acid sequence as shown in SEQ ID NO: 2; or
a salt of (a) or (b).

SEQUENCE LISTING

<110>  YOKOHAMA CITY UNIVERSITY

<120>  TRF2 DNA-binding domain mutant proteins, telomeric DNA mutants, and
use of a structure of a complex between a TRF2 DNA binding domain and a
double-stranded DNA molecule

<130>  N.98807 GCW

<140>  EP 05719579.4
<141>  2005-02-21

<150>  PCT/JP2005/03237
<151>  2005-02-21

<150>  JP P2004-046238
<151>  2004-02-23

<160>  29

<170>  PatentIn version 3.1

<210>  1
<211>  189
<212>  DNA
<213>  Homo sapiens

<220>
<221>  CDS
<222>  (1)..(189)
<223>

<400>  1
gaa gac agt aca acc aat ata aca aaa aag cag aag tgg act gta gaa        48
Glu Asp Ser Thr Thr Asn Ile Thr Lys Lys Gln Lys Trp Thr Val Glu
1               5                   10                  15

gaa agc gag tgg gtc aag gct gga gtg cag aaa tat ggg gaa gga aac        96
Glu Ser Glu Trp Val Lys Ala Gly Val Gln Lys Tyr Gly Glu Gly Asn
            20                  25                  30

tgg gct gcc att tct aaa aat tac cca ttt gtt aac cga aca gct gtg        144
Trp Ala Ala Ile Ser Lys Asn Tyr Pro Phe Val Asn Arg Thr Ala Val
        35                  40                  45

atg att aag gat cgc tgg cgg acc atg aaa aga ctt ggc atg aac            189
Met Ile Lys Asp Arg Trp Arg Thr Met Lys Arg Leu Gly Met Asn
    50                  55                  60


<210>  2
<211>  63
<212>  PRT
<213>  Homo sapiens

<400>  2

Glu Asp Ser Thr Thr Asn Ile Thr Lys Lys Gln Lys Trp Thr Val Glu
1               5                   10                  15


Glu Ser Glu Trp Val Lys Ala Gly Val Gln Lys Tyr Gly Glu Gly Asn
            20                  25                  30


Trp Ala Ala Ile Ser Lys Asn Tyr Pro Phe Val Asn Arg Thr Ala Val
        35                  40                  45

Met Ile Lys Asp Arg Trp Arg Thr Met Lys Arg Leu Gly Met Asn
    50                  55                  60

<210>  3
<211>  189
<212>  DNA
<213>  Artificial

<220>
<223>  synthetic DNA

<220>
<221>  CDS
<222>  (1)..(189)
<223>

<400>  3
gaa gac agt aca acc aat ata aca aaa agg cag aag tgg act gta gaa        48
Glu Asp Ser Thr Thr Asn Ile Thr Lys Arg Gln Lys Trp Thr Val Glu
1               5                   10                  15

gaa agc gag tgg gtc aag gct gga gtg cag aaa tat ggg gaa gga aac        96
Glu Ser Glu Trp Val Lys Ala Gly Val Gln Lys Tyr Gly Glu Gly Asn
            20                  25                  30

tgg gct gcc att tct aaa aat tac cca ttt gtt aac cga aca gct gtg       144
Trp Ala Ala Ile Ser Lys Asn Tyr Pro Phe Val Asn Arg Thr Ala Val
        35                  40                  45

atg att aag gat cgc tgg cgg acc atg aaa aga ctt ggc atg aac           189
Met Ile Lys Asp Arg Trp Arg Thr Met Lys Arg Leu Gly Met Asn
    50                  55                  60

<210>  4
<211>  63
<212>  PRT
<213>  Artificial

<220>
<223>  synthetic DNA

<400>  4

Glu Asp Ser Thr Thr Asn Ile Thr Lys Arg Gln Lys Trp Thr Val Glu
1               5                   10                  15

Glu Ser Glu Trp Val Lys Ala Gly Val Gln Lys Tyr Gly Glu Gly Asn
            20                  25                  30

Trp Ala Ala Ile Ser Lys Asn Tyr Pro Phe Val Asn Arg Thr Ala Val
        35                  40                  45

Met Ile Lys Asp Arg Trp Arg Thr Met Lys Arg Leu Gly Met Asn
    50                  55                  60

<210>  5
<211>  189
<212>  DNA
<213>  Artificial

<220>
<223>  synthetic DNA

<220>
<221>  CDS
<222>  (1)..(189)
<223>

<400>  5
gaa gac agt aca acc aat ata aca aaa aag cag aag tgg act gta gaa        48
Glu Asp Ser Thr Thr Asn Ile Thr Lys Lys Gln Lys Trp Thr Val Glu
1               5                   10                  15

gaa agc gag tgg gtc aag gct gga gtg cag aaa tat ggg gaa gga aac        96
Glu Ser Glu Trp Val Lys Ala Gly Val Gln Lys Tyr Gly Glu Gly Asn
            20                  25                  30

tgg tct gcc att tct aaa aat tac cca ttt gtt aac cga aca gct gtg        144
Trp Ser Ala Ile Ser Lys Asn Tyr Pro Phe Val Asn Arg Thr Ala Val
        35                  40                  45

atg att aag gat cgc tgg cgg acc atg aaa aga ctt ggc atg aac            189
Met Ile Lys Asp Arg Trp Arg Thr Met Lys Arg Leu Gly Met Asn
    50                  55                  60


<210>  6
<211>  63
<212>  PRT
<213>  Artificial

<220>
<223>  synthetic DNA

<400>  6

Glu Asp Ser Thr Thr Asn Ile Thr Lys Lys Gln Lys Trp Thr Val Glu
1               5                   10                  15


Glu Ser Glu Trp Val Lys Ala Gly Val Gln Lys Tyr Gly Glu Gly Asn
            20                  25                  30


Trp Ser Ala Ile Ser Lys Asn Tyr Pro Phe Val Asn Arg Thr Ala Val
        35                  40                  45


Met Ile Lys Asp Arg Trp Arg Thr Met Lys Arg Leu Gly Met Asn
    50                  55                  60


<210>  7
<211>  189
<212>  DNA
<213>  Artificial

<220>
<223>  synthetic DNA

<220>
<221>  CDS
<222>  (1)..(189)
<223>

<400>  7
gaa gac agt aca acc aat ata aca aaa aag cag aag tgg act gta gaa        48
Glu Asp Ser Thr Thr Asn Ile Thr Lys Lys Gln Lys Trp Thr Val Glu
1               5                   10                  15

gaa agc gag tgg gtc aag gct gga gtg cag aaa tat ggg gaa gga aac        96

Glu Ser Glu Trp Val Lys Ala Gly Val Gln Lys Tyr Gly Glu Gly Asn
            20                  25                  30

tgg gct gcc att tct aaa aat tac cca ttt gtt aac cga aca tct gtg        144
Trp Ala Ala Ile Ser Lys Asn Tyr Pro Phe Val Asn Arg Thr Ser Val
        35                  40                  45

atg att aag gat cgc tgg cgg acc atg aaa aga ctt ggc atg aac            189
Met Ile Lys Asp Arg Trp Arg Thr Met Lys Arg Leu Gly Met Asn
    50                  55                  60

<210>  8
<211>  63
<212>  PRT
<213>  Artificial

<220>
<223>  synthetic DNA

<400>  8

Glu Asp Ser Thr Thr Asn Ile Thr Lys Lys Gln Lys Trp Thr Val Glu
1               5                   10                  15

Glu Ser Glu Trp Val Lys Ala Gly Val Gln Lys Tyr Gly Glu Gly Asn
            20                  25                  30

Trp Ala Ala Ile Ser Lys Asn Tyr Pro Phe Val Asn Arg Thr Ser Val
        35                  40                  45

Met Ile Lys Asp Arg Trp Arg Thr Met Lys Arg Leu Gly Met Asn
    50                  55                  60

<210>  9
<211>  189
<212>  DNA
<213>  Artificial

<220>
<223>  synthetic DNA

<220>
<221>  CDS
<222>  (1)..(189)
<223>

<400>  9
gaa gac agt aca acc aat ata aca aaa aag cag aag tgg act gta gaa         48
Glu Asp Ser Thr Thr Asn Ile Thr Lys Lys Gln Lys Trp Thr Val Glu
1               5                   10                  15

gaa agc gag tgg gtc aag gct gga gtg cag aaa tat ggg gaa gga aac         96
Glu Ser Glu Trp Val Lys Ala Gly Val Gln Lys Tyr Gly Glu Gly Asn
            20                  25                  30

tgg gct gcc att tct aaa aat tac cca ttt gtt aac cga aca gct gtg        144
Trp Ala Ala Ile Ser Lys Asn Tyr Pro Phe Val Asn Arg Thr Ala Val
        35                  40                  45

atg att aag gat cgc tgg cgg acc atg aaa aag ctt ggc atg aac            189
Met Ile Lys Asp Arg Trp Arg Thr Met Lys Lys Leu Gly Met Asn
    50                  55                  60

<210>  10
<211>  63
<212>  PRT
<213>  Artificial

<220>
<223>  synthetic DNA

<400>  10

Glu Asp Ser Thr Thr Asn Ile Thr Lys Lys Gln Lys Trp Thr Val Glu
1               5                   10                  15

Glu Ser Glu Trp Val Lys Ala Gly Val Gln Lys Tyr Gly Glu Gly Asn
            20                  25                  30

Trp Ala Ala Ile Ser Lys Asn Tyr Pro Phe Val Asn Arg Thr Ala Val
        35                  40                  45

Met Ile Lys Asp Arg Trp Arg Thr Met Lys Lys Leu Gly Met Asn
    50                  55                  60

<210>  11
<211>  189
<212>  DNA
<213>  Artificial

<220>
<223>  synthetic DNA

<220>
<221>  CDS
<222>  (1)..(189)
<223>

<400>  11
gaa gac agt aca acc aat ata aca aaa agg cag aag tgg act gta gaa      48
Glu Asp Ser Thr Thr Asn Ile Thr Lys Arg Gln Lys Trp Thr Val Glu
1               5                   10                  15

gaa agc gag tgg gtc aag gct gga gtg cag aaa tat ggg gaa gga aac      96
Glu Ser Glu Trp Val Lys Ala Gly Val Gln Lys Tyr Gly Glu Gly Asn
            20                  25                  30

tgg tct gcc att tct aaa aat tac cca ttt gtt aac cga aca tct gtg      144
Trp Ser Ala Ile Ser Lys Asn Tyr Pro Phe Val Asn Arg Thr Ser Val
        35                  40                  45

atg att aag gat cgc tgg cgg acc atg aaa aag ctt ggc atg aac          189
Met Ile Lys Asp Arg Trp Arg Thr Met Lys Lys Leu Gly Met Asn
    50                  55                  60

<210>  12
<211>  63
<212>  PRT
<213>  Artificial

<220>
<223>  synthetic DNA

<400>  12

Glu Asp Ser Thr Thr Asn Ile Thr Lys Arg Gln Lys Trp Thr Val Glu
1               5                   10                  15

Glu Ser Glu Trp Val Lys Ala Gly Val Gln Lys Tyr Gly Glu Gly Asn
            20                  25                  30

Trp Ser Ala Ile Ser Lys Asn Tyr Pro Phe Val Asn Arg Thr Ser Val
        35                  40                  45

Met Ile Lys Asp Arg Trp Arg Thr Met Lys Lys Leu Gly Met Asn
    50                  55                  60

<210>  13
<211>  189
<212>  DNA
<213>  Artificial

<220>
<223>  synthetic DNA

<220>
<221>  CDS
<222>  (1)..(189)
<223>

<400>  13
gaa gac agt aca acc aat ata aca aaa aag cag aag tgg act gta gaa        48
Glu Asp Ser Thr Thr Asn Ile Thr Lys Lys Gln Lys Trp Thr Val Glu
1               5                   10                  15

gaa agc gag tgg gtc aag gct gga gtg cag aaa tat ggg gaa gga aac        96
Glu Ser Glu Trp Val Lys Ala Gly Val Gln Lys Tyr Gly Glu Gly Asn
            20                  25                  30

tgg tct gcc att tct aaa aat tac cca ttt gtt aac cga aca tct gtg       144
Trp Ser Ala Ile Ser Lys Asn Tyr Pro Phe Val Asn Arg Thr Ser Val
        35                  40                  45

atg att aag gat cgc tgg cgg acc atg aaa aga ctt ggc atg aac           189
Met Ile Lys Asp Arg Trp Arg Thr Met Lys Arg Leu Gly Met Asn
    50                  55                  60

<210>  14
<211>  63
<212>  PRT
<213>  Artificial

<220>
<223>  synthetic DNA

<400>  14

Glu Asp Ser Thr Thr Asn Ile Thr Lys Lys Gln Lys Trp Thr Val Glu
1               5                   10                  15

Glu Ser Glu Trp Val Lys Ala Gly Val Gln Lys Tyr Gly Glu Gly Asn
            20                  25                  30

Trp Ser Ala Ile Ser Lys Asn Tyr Pro Phe Val Asn Arg Thr Ser Val
        35                  40                  45

Met Ile Lys Asp Arg Trp Arg Thr Met Lys Arg Leu Gly Met Asn
    50                  55                  60

<210> 15
<211> 1500
<212> DNA
<213> Homo sapiens

<220>
<221> CDS
<222> (1)..(1500)
<223>

<400> 15
atg gcg gga gga ggc ggg agt agc gac ggc agc ggg cgg gca gct ggc 48
Met Ala Gly Gly Gly Gly Ser Ser Asp Gly Ser Gly Arg Ala Ala Gly
1               5                   10                  15

agg cgg gcg tcc cgc agt agc ggg cgg gcc cgg cgg ggg cgc cac gag 96
Arg Arg Ala Ser Arg Ser Ser Gly Arg Ala Arg Arg Gly Arg His Glu
            20                  25                  30

ccg ggg ctg ggg ggc ccg gcg gag cgc ggc gcg ggg gag gca cgg ctg 144
Pro Gly Leu Gly Gly Pro Ala Glu Arg Gly Ala Gly Glu Ala Arg Leu
        35                  40                  45

gaa gag gca gtc aat cgc tgg gtg ctc aag ttc tac ttc cac gag gcg 192
Glu Glu Ala Val Asn Arg Trp Val Leu Lys Phe Tyr Phe His Glu Ala
    50                  55                  60

ctg cgg gcc ttt cgg ggt agc cgg tac ggg gac ttc aga cag atc cgg 240
Leu Arg Ala Phe Arg Gly Ser Arg Tyr Gly Asp Phe Arg Gln Ile Arg
65                  70                  75                  80

gac atc atg cag gct ttg ctt gtc agg ccc ttg ggg aag gag cac acc 288
Asp Ile Met Gln Ala Leu Leu Val Arg Pro Leu Gly Lys Glu His Thr
                85                  90                  95

gtg tcc cga ttg ctg cgg gtt atg cag tgt ctg tcg cgg att gaa gaa 336
Val Ser Arg Leu Leu Arg Val Met Gln Cys Leu Ser Arg Ile Glu Glu
            100                 105                 110

ggg gaa aat tta gac tgt tcc ttt gat atg gag gct gag ctc aca cca 384
Gly Glu Asn Leu Asp Cys Ser Phe Asp Met Glu Ala Glu Leu Thr Pro
        115                 120                 125

ctg gaa tca gct atc aat gtg ctg gag atg att aaa acg gaa ttt aca 432
Leu Glu Ser Ala Ile Asn Val Leu Glu Met Ile Lys Thr Glu Phe Thr
    130                 135                 140

ctg aca gaa gca gtg gtc gaa tcc agt aga aaa ctg gtc aag gaa gct 480
Leu Thr Glu Ala Val Val Glu Ser Ser Arg Lys Leu Val Lys Glu Ala
145                 150                 155                 160

gct gtc att att tgt atc aaa aac aaa gaa ttt gaa aag gct tca aaa 528
Ala Val Ile Ile Cys Ile Lys Asn Lys Glu Phe Glu Lys Ala Ser Lys
                165                 170                 175

att ttg aaa aaa cat atg tcc aag gac ccc aca act cag aag ctg aga 576
Ile Leu Lys Lys His Met Ser Lys Asp Pro Thr Thr Gln Lys Leu Arg
            180                 185                 190

aat gat ctc ctg aat att att cga gaa aag aac ttg gcc cat cct gtt 624
Asn Asp Leu Leu Asn Ile Ile Arg Glu Lys Asn Leu Ala His Pro Val
        195                 200                 205

atc cag aac ttt tca tat gag acc ttc cag cag aag atg ctg cgc ttc 672
Ile Gln Asn Phe Ser Tyr Glu Thr Phe Gln Gln Lys Met Leu Arg Phe
    210                 215                 220

ctg gag agc cac ctg gat gac gcc gag ccc tac ctc ctc acg atg gcc 720
Leu Glu Ser His Leu Asp Asp Ala Glu Pro Tyr Leu Leu Thr Met Ala
225                 230                 235                 240

aaa aag gct ttg aaa tct gag tcc gct gcc tca agt aca ggg aag gaa 768
Lys Lys Ala Leu Lys Ser Glu Ser Ala Ala Ser Ser Thr Gly Lys Glu
                245                 250                 255

gat aaa cag cca gca cca ggg cct gtg gaa aag cca ccc aga gaa ccc 816
Asp Lys Gln Pro Ala Pro Gly Pro Val Glu Lys Pro Pro Arg Glu Pro
            260                 265                 270

gca agg cag cta cgg aat cct cca acc acc att gga atg atg act ctg 864
Ala Arg Gln Leu Arg Asn Pro Pro Thr Thr Ile Gly Met Met Thr Leu
        275                 280                 285

aaa gca gct ttc aag act ctg tct ggt gca cag gat tct gag gca gcc 912
Lys Ala Ala Phe Lys Thr Leu Ser Gly Ala Gln Asp Ser Glu Ala Ala
    290                 295                 300

ttt gca aaa ctg gac cag aag gat ctg gtt ctt cct act caa gct ctc 960
Phe Ala Lys Leu Asp Gln Lys Asp Leu Val Leu Pro Thr Gln Ala Leu
305                 310                 315                 320

cca gca tca cca gcc ctc aaa aac aag aga ccc aga aaa gat gaa aac 1008
Pro Ala Ser Pro Ala Leu Lys Asn Lys Arg Pro Arg Lys Asp Glu Asn
                325                 330                 335

gaa agt tca gcc ccg gct gac ggt gag ggt ggc tcg gaa ctg cag ccc 1056
Glu Ser Ser Ala Pro Ala Asp Gly Glu Gly Gly Ser Glu Leu Gln Pro
            340                 345                 350

aag aac aag cgc atg aca ata agc aga ttg gtc ttg gag gag gac agc 1104
Lys Asn Lys Arg Met Thr Ile Ser Arg Leu Val Leu Glu Glu Asp Ser
        355                 360                 365

cag agt act gag ccc agc gca ggc ctc aac tcc tcc cag gag gcc gct 1152
Gln Ser Thr Glu Pro Ser Ala Gly Leu Asn Ser Ser Gln Glu Ala Ala
    370                 375                 380

tca gcg cca cca tcc aag ccc acc gtt ctc aac caa ccc ctc cct gga 1200
Ser Ala Pro Pro Ser Lys Pro Thr Val Leu Asn Gln Pro Leu Pro Gly
385                 390                 395                 400

gag aag aat ccc aaa gta ccc aaa ggc aag tgg aac agc tct aat ggg 1248
Glu Lys Asn Pro Lys Val Pro Lys Gly Lys Trp Asn Ser Ser Asn Gly
                405                 410                 415

gtt gaa gaa aag gag act tgg gtg gaa gag gat gaa ctg ttt caa gtt 1296
Val Glu Glu Lys Glu Thr Trp Val Glu Glu Asp Glu Leu Phe Gln Val
            420                 425                 430

cag gca gca cca gat gaa gac agt aca acc aat ata aca aaa aag cag 1344
Gln Ala Ala Pro Asp Glu Asp Ser Thr Thr Asn Ile Thr Lys Lys Gln
        435                 440                 445

aag tgg act gta gaa gaa agc gag tgg gtc aag gct gga gtg cag aaa 1392
Lys Trp Thr Val Glu Glu Ser Glu Trp Val Lys Ala Gly Val Gln Lys
    450                 455                 460

tat ggg gaa gga aac tgg gct gcc att tct aaa aat tac cca ttt gtt 1440
Tyr Gly Glu Gly Asn Trp Ala Ala Ile Ser Lys Asn Tyr Pro Phe Val
465                 470                 475                 480

aac cga aca gct gtg atg att aag gat cgc tgg cgg acc atg aaa aga 1488
Asn Arg Thr Ala Val Met Ile Lys Asp Arg Trp Arg Thr Met Lys Arg
                485                 490                 495

ctt ggc atg aac 1500

Leu Gly Met Asn
            500

<210>  16
<211>  500
<212>  PRT
<213>  Homo sapiens

<400>  16

Met Ala Gly Gly Gly Gly Ser Ser Asp Gly Ser Gly Arg Ala Ala Gly
1               5                   10                  15

Arg Arg Ala Ser Arg Ser Ser Gly Arg Ala Arg Arg Gly Arg His Glu
            20                  25                  30

Pro Gly Leu Gly Gly Pro Ala Glu Arg Gly Ala Gly Glu Ala Arg Leu
        35                  40                  45

Glu Glu Ala Val Asn Arg Trp Val Leu Lys Phe Tyr Phe His Glu Ala
    50                  55                  60

Leu Arg Ala Phe Arg Gly Ser Arg Tyr Gly Asp Phe Arg Gln Ile Arg
65                  70                  75                  80

Asp Ile Met Gln Ala Leu Leu Val Arg Pro Leu Gly Lys Glu His Thr
                85                  90                  95

Val Ser Arg Leu Leu Arg Val Met Gln Cys Leu Ser Arg Ile Glu Glu
            100                 105                 110

Gly Glu Asn Leu Asp Cys Ser Phe Asp Met Glu Ala Glu Leu Thr Pro
        115                 120                 125

Leu Glu Ser Ala Ile Asn Val Leu Glu Met Ile Lys Thr Glu Phe Thr
    130                 135                 140

Leu Thr Glu Ala Val Val Glu Ser Ser Arg Lys Leu Val Lys Glu Ala
145                 150                 155                 160

Ala Val Ile Ile Cys Ile Lys Asn Lys Glu Phe Glu Lys Ala Ser Lys
                165                 170                 175

Ile Leu Lys Lys His Met Ser Lys Asp Pro Thr Thr Gln Lys Leu Arg
            180                 185                 190

Asn Asp Leu Leu Asn Ile Ile Arg Glu Lys Asn Leu Ala His Pro Val
        195                 200                 205

Ile Gln Asn Phe Ser Tyr Glu Thr Phe Gln Gln Lys Met Leu Arg Phe
    210                 215                 220

Leu Glu Ser His Leu Asp Asp Ala Glu Pro Tyr Leu Leu Thr Met Ala
225                 230                 235                 240

Lys Lys Ala Leu Lys Ser Glu Ser Ala Ala Ser Ser Thr Gly Lys Glu
                245                 250                 255

Asp Lys Gln Pro Ala Pro Gly Pro Val Glu Lys Pro Pro Arg Glu Pro
            260                 265                 270

Ala Arg Gln Leu Arg Asn Pro Pro Thr Thr Ile Gly Met Met Thr Leu
        275                 280                 285

Lys Ala Ala Phe Lys Thr Leu Ser Gly Ala Gln Asp Ser Glu Ala Ala
    290                 295                 300

Phe Ala Lys Leu Asp Gln Lys Asp Leu Val Leu Pro Thr Gln Ala Leu
305                 310                 315                 320

Pro Ala Ser Pro Ala Leu Lys Asn Lys Arg Pro Arg Lys Asp Glu Asn
                325                 330                 335

Glu Ser Ser Ala Pro Ala Asp Gly Glu Gly Gly Ser Glu Leu Gln Pro
            340                 345                 350

Lys Asn Lys Arg Met Thr Ile Ser Arg Leu Val Leu Glu Glu Asp Ser
        355                 360                 365

Gln Ser Thr Glu Pro Ser Ala Gly Leu Asn Ser Ser Gln Glu Ala Ala
    370                 375                 380

Ser Ala Pro Pro Ser Lys Pro Thr Val Leu Asn Gln Pro Leu Pro Gly
385                 390                 395                 400

Glu Lys Asn Pro Lys Val Pro Lys Gly Lys Trp Asn Ser Ser Asn Gly
                405                 410                 415

Val Glu Glu Lys Glu Thr Trp Val Glu Glu Asp Glu Leu Phe Gln Val
            420                 425                 430

Gln Ala Ala Pro Asp Glu Asp Ser Thr Thr Asn Ile Thr Lys Lys Gln
        435                 440                 445

Lys Trp Thr Val Glu Glu Ser Glu Trp Val Lys Ala Gly Val Gln Lys
    450                 455                 460

Tyr Gly Glu Gly Asn Trp Ala Ala Ile Ser Lys Asn Tyr Pro Phe Val
465                 470                 475                 480

Asn Arg Thr Ala Val Met Ile Lys Asp Arg Trp Arg Thr Met Lys Arg
                485                 490                 495

Leu Gly Met Asn
            500

<210>  17
<211>  13
<212>  DNA
<213>  Artificial

<220>
<223>  synthetic DNA

<400>  17
gttagggtta ggg                                                          13


<210>  18
<211>  13
<212>  DNA
<213>  Artificial

<220>
<223>  synthetic DNA

<400>  18
ccctaaccct aac                                                          13


<210>  19
<211>  13
<212>  DNA
<213>  Artificial

<220>
<223>  synthetic DNA

<400>  19
gtgagggtta ggg                                                          13


<210>  20
<211>  13
<212>  DNA
<213>  Artificial

<220>
<223>  synthetic DNA

<400>  20
gttaggctta ggg                                                          13


<210>  21
<211>  13
<212>  DNA
<213>  Artificial

<220>
<223>  synthetic DNA

<400>  21
gttagggtga ggg                                                          13


<210>  22
<211>  36
<212>  DNA
<213>  Artificial

<220>
<223>  synthetic DNA

<400>  22
ggtctcgcat atggaagaca gtacaaccaa tataac                                36

<210>  23
<211>  32
<212>  DNA
<213>  Artificial

<220>
<223>  synthetic DNA

<400>  23
gcgggaattc tcagttcatg ccaagtcttt tc                                    32

<210>  24
<211>  27
<212>  DNA
<213>  Artificial

<220>
<223>  synthetic DNA

<400>  24
ggaaactggt ctgccatttc taaaaat                                          27

<210>  25
<211>  27
<212>  DNA
<213>  Artificial

<220>
<223>  synthetic DNA

<400>  25
agaaatggca gaccagtttc cttcccc                                          27

<210>  26
<211>  27
<212>  DNA
<213>  Artificial

<220>
<223>  synthetic DNA

<400>  26
aaccgaacat ctgtgatgat taaggat                                          27

<210>  27
<211>  27
<212>  DNA
<213>  Artificial

<220>
<223>  synthetic DNA

<400>  27
aatcatcaca gatgttcggt taacaaa                                          27

<210>  28
<211>  52
<212>  DNA
<213>  Artificial

<220>
<223>  synthetic DNA

<400>  28
ggtctcgcat atggaagaca gtacaaccaa tataacaaaa aggcagaagt gg          52


<210>  29
<211>  37
<212>  DNA
<213>  Artificial

<220>
<223>  synthetic DNA

<400>  29
ggaattctca gttcatgcca agttttttca tggtccg          37

## Description

### Technical Field

The present invention relates to telomeric protein TRF2 DNA-binding domain mutant proteins, telomeric DNA mutants, and use of the complex structure composed of TRF2 DNA-binding domain and duplex DNA.

### Background Art

Chromosomal ends in vertebrates, which are called telomeres, are composed of GG sequence-rich repetitive DNA and proteins. Ends of mammalian telomeres are composed of telomeric DNA-binding proteins, TRF1 and TRF2, and the other proteins which interact with TRF 1 or TRF2, and escape recognition by the watch mechanism for damaged ends of double stranded DNA (non-patent documents 1-3). Recently, a great number of reports supporting that telomeric protein TRF2 plays an important role in the formation of a functional telomeric structure have been presented. TRF2 consists of 500 amino acids in full length with a dimerization domain consisting of 420 residues at its N-terminus and a DNA-binding domain consisting of 60 residues at its C-terminus. Telomeric DNA-binding protein TRF1, which is highly homologous to TRF2, consists of 439 amino acids with a dimerization domain at its N-terminus and a DNA-binding domain at its C-terminus. Although the corresponding domains have very high homology to each other, about 50 amino acids before the dimerization domain are rich in basic amino acids in TRF2 whereas they are rich in acidic amino acids in TRF1 (non-patent documents 2-5). The DNA-binding domains of TRF1 and TRF2 recognize the same DNA sequence, tandem repeat sequence of TTAGGG.. TRF1 recognizes the double-stranded DNA sequence and bends the DNA *in vitro* (non-patent documents 4, 6 and 7), however TRF2 preferentially binds to the junction between the double-stranded DNA and the 3'-overhang to allow t-loop formation (non-patent document 8). The "t-loop" is a loop structure formed by the 3'-overhang invading the telomeric sequence of the inner strand of the double stranded DNA. Within the t-loop TRF2 is bound to the junction (non-patent document 9). Inhibition of the TRF2 binding to the telomeric DNA immediately induces instability of chromosomes, senescence mediated by p16/RB system, and cell death by apoptosis mediated by ATM/p53 system (non-patent documents 10-13). In other words, DNA binding modes of TRF1 and TRF2 are greatly different, and it is believed that telomere-binding molecules do not simply bind to the telomeric sequence but form a functional telomeric structure by causing a three-dimensional structural change at the telomeric end (non-patent document 9). The DNA-binding domains of TRF1 and TRF2 have a high homology of about 60% and recognize the same sequence of double-stranded DNA. It is known that the sequences of the DNA-binding domains of TRF1 and TRF2 are highly homologous to the three repeat sequences in the DNA-binding domain of a protooncogene product c-Myb (non-patent documents 14 and 15). The DNA-binding domain of c-Myb consists of three tandem repeats, designated as R1, R2 and R3, each consisting of 53 amino acid residues. Each repeat contains three helices. Like the c-Myb DNA-binding repeat, the DNA-binding domain of TRF1 (which was reported previously) consists of three helices, and the third helix contacts in the major groove of telomeric DNA deeply to thereby bind the DNA (non-patent document 16). In the DNA-binding domain of TRF2, the amino acids involved in DNA recognition in TRF1 are almost conserved. Thus, it is believed that TRF2 has a DNA binding mode similar to that of TRF1. To examine the structure and nature of TRF2-DNA complex and elucidate the difference between TRF2 and TRF1 are very important for examining the chromosome end protection mechanism of telomeres.
[Non-patent document 1]
   Bilaud, T., Brun, C., Ancelin, K., Koering, C.E., Laroche, T. & Gilson, E. Telomeric localization of TRF2, a novel human telobox protein. Nat. Genet. 17, 236-239. (1997)
[Non-patent document 2]
   Broccoli, D., Smogorzewska, A., Chong, L. & de Lange, T. Human telomeres contain two distinct Myb-related proteins, TRF1 and TRF2. Nat. Genet. 17, 231-235. (1997)
[Non-patent document 3]
   Chong, L., van Steensel, B., Broccoli, D., Erdjument-Bromage, H., Hanish, J., Tempst, P. & de Lange, T. A human telomeric protein. Science 270, 1663-1667. (1995)
[Non-patent document 4]
   Bianchi, A., Smith, S., Chong, L., Elias, P. & de Lange, T. TRF1 is a dimer and bends telomeric DNA. EMBO J. 16, 1785-1794. (1997)
[Non-patent document 5]
   Smith, S. & de Lange, T. TRF1, a mammalian telomeric protein. Trends Genet. 13, 21-26. (1997)
[Non-patent document 6]
   Bianchi, A.M., Stansel, R.M., Fairall, L.D., Griffith, J.D., Rhodes, D. & de Lange, T. TRF1 binds a bipartite telomeric site with extreme spatial flexibility. EMBO J. 18, 5735-5744. (1999)
[Non-patent document 7]
   Griffith, J., Bianchi, A. & de Lange, T. TRF1 promotes parallel pairing of telomeric tracts in vitro. J. Mol. Biol. 278, 79-88. (1998)
[Non-patent document 8]
   Stansel, R.M., de Lange, T. & Griffith, J.D. T-loop assembly in vitro involves binding of TRF2 near the 3' telomeric overhang. EMBO J. 20, E5532-E5540. (2001)
[Non-patent document 9]
   Griffith, J.D., Comenau, L., Rosenfield, S., Stansel, R.M., Bianch, A., Moss, H. & de Lange, T. Mammalian telomeres end in a large duplex loop. Cell 97, 503-514. (1999)
[Non-patent document 10]
   Karlseder, J., Smogorzewska, A. & de Lange, T. Senesecence induced by altered telomere state, not telomere loss. Science 295, 2446-2449. (2002)
[Non-patent document 11]
   van Steensel, B., Smogorzewska, A. & de Lange, T. TRF2 protects human telomeres from end-to-end fusion. Cell 92, 401-413. (1998)
[Non-patent document 12]
   Smogorzewska, A. & de Lange, T. Different telomere damage signaling pathways in human and mouse cells, EMBO J. 21, 4338-4348. (2002)
[Non-patent document 13]
   Kalseder, J., Broccoli, D., Dai, Y., Hardy, S. & de Lange, T. p53- and ATM-dependent apoptosis induced by telomeres lacking TRF2. Science 283, 1321-1325. (1999)
[Non-patent document 14]
   Gonda, T.J., Gough, N.M., Dunn, A.R. & de Blaquiere, J. Nucleotide sequence of cDNA clones of the murine myb proto-oncogene. EMBO J. 4, 2003-2008. (1985)
[Non-patent document 15]
   Klempnauer, K.H. & Sippel, A.E. The highly conserved amino-terminal region of the protein encoded by the v-myb oncogene functions as a DNA-binding domain. EMBO J 6, 2719-2725. (1987)
[Non-patent document 16]
   Nishikawa, T., Okamura, H., Nagadoi, A., Konig, P., Rhodes, D. & Nishimura, Y. Solution structure of a telomere DNA complex of human TRF1. Strucure 9, 1237-1251. (2001)

It is an object of the invention to analyze the structure and function of the complex composed of the telomeric protein TRF2 DNA-binding domain and telomeric duplex DNA.

Further, it is another object of the invention to enable screening for drugs capable of regulating the DNA-binding ability of TRF2 and to contribute to drug discovery targeting TRF2, based on the results of the above analyses.

### Disclosure of Invention

The present inventors determined the structure of the complex composed of the telomeric protein TRF2 DNA-binding domain and telomeric duplex DNA by NMR. The inventors compared the modes of recognition of duplex DNA by TRF2 and TRF1 using the previously reported complex composed of the telomeric protein TRF1 DNA-binding domain and telomeric duplex DNA (Nishikawa, T., Okamura, H., Nagadoi, A., Konig, P., Rhodes, D. & Nishimura, Y. Solution structure of a telomere DNA complex of human TRF1. Strucure 9, 1237-1251. (2001)). Although amino acid sequences of the TRF1 and TRF2 DNA-binding domains are different about 40% from each other (their homology is about 60%), the inventors have succeeded as a result of structural comparison in converting the DNA-binding ability of TRF2 to a TRF1-type ability by replacing only four amino acid residues in TRF2 with the corresponding amino acid residues in TRF1. That is, the inventors could prepare a TRF2 mutant which has a much stronger binding ability to telomeric duplex DNA than the wild-type TRF2. The present invention has been achieved based on these findings.

The summary of the present invention is as follows.
(1) A TRF2 DNA-binding domain mutant protein comprising:
   (a) a TRF2 DNA-binding domain mutant protein having an amino acid sequence as shown in SEQ ID NO: 2 but with at least one substitution selected from the group consisting of substitution of the lysine residue with arginine at position 10, substitution of the alanine residue with serine at position 34, substitution of the alanine residue with serine at position 47 and substitution of the arginine residue with lysine at position 59; or
   (b) a TRF2 DNA-binding domain mutant protein having an amino acid sequence of the mutant protein of (a) above but with one or several amino acid residues other than.the amino acid residues at positions 10, 34, 47 and 59 being deleted, substituted or added, and which has a higher binding ability to a duplex DNA comprising a sequence represented by 5'-TTAGGG-3' than a wild-type TRF2 DNA-binding domain protein having an amino acid sequence as shown in SEQ ID NO: 2; or a salt of (a) or (b).
(2) The TRF2 DNA-binding domain mutant protein or a salt thereof of (1) above, wherein the mutant protein of (a) is any one of the following (ia) to (via):
   (ia) a protein having an amino acid sequence as shown in SEQ ID NO: 2 but with the lysine residue at position 10 being substituted with arginine;
   (iia) a protein having an amino acid sequence as shown in SEQ ID NO: 2 but with the alanine residue at position 34 being substituted with serine;
   (iiia) a protein having an amino acid sequence as shown in SEQ ID NO: 2 but with the alanine residue at position 47 being substituted with serine;
   (iva) a protein having an amino acid sequence as shown in SEQ ID NO: 2 but with the arginine residue at position 59 being substituted with lysine;
   (va) a protein having an amino acid sequence as shown in SEQ ID NO: 2 but with the lysine residue at position 10 being substituted with arginine, the alanine residue at position 34 being substituted with serine, the alanine residue at position 47 being substituted with serine and the arginine residue at position 59 being substituted with lysine;
   (via) a protein having an amino acid sequence as shown in SEQ ID NO: 2 but with the lysine residue at position 10 being substituted with arginine and the alanine residue at position 47 being substituted with serine;
   (viia) a protein having an amino acid sequence as shown in SEQ ID NO: 2 but with the alanine residue at position 34 being substituted with serine and the alanine residue at position 47 being substituted with serine; or
   (viiia) a protein having an amino acid sequence as shown in SEQ ID NO: 2 but with the lysine residue at position 10 being substituted with arginine, the alanine residue at position 34 being substituted with serine and the alanine residue at position 47 being substituted with serine.
(3) An isolated DNA encoding the protein of (1) above.
(4) A recombinant vector comprising the DNA of (3) above.
(5) A transformant comprising the recombinant vector of (4) above.
(6) A method of producing a TRF2 DNA-binding domain mutant protein, comprising culturing a host transformed with the DNA of (3) above and recovering the TRF2 DNA-binding domain mutant protein from the resultant culture.
(7) An antibody to the TRF2 DNA-binding domain mutant protein or a salt thereof of (1) above.
(8) A protein comprising the TRF2 DNA-binding domain mutant protein of (1) above; or a salt thereof.
(9) A complex of the protein of (1) or (8) above and a DNA.
(10) A DNA having a nucleotide sequence as shown in SEQ ID NO: 17 but with at least one substitution selected from the group consisting of substitution of the T at position 3 with G, substitution of the G at position 7 to C and substitution of the T at position 9 to G.
(11) The DNA of (10) above, which is any one of the following (ib) to (iiib):
   (ib) a DNA having a nucleotide sequence as shown in SEQ ID NO: 17 with the T at position 3 being substituted with G;
   (iib) a DNA having a nucleotide sequence as shown in SEQ ID NO: 17 with the G at position 7 being substituted with C; or
   (iiib) a DNA having a nucleotide sequence as shown in SEQ ID NO: 17 with the T at position 9 being substituted with G.
(12) A method of screening for substances which are capable of regulating the binding of telomeric DNA to TRF2, comprising analyzing whether or not a TRF2 DNA-binding domain having an amino acid sequence as shown in SEQ ID NO: 2 or a protein comprising the domain interacts with a test substance at least at one amino acid site selected from the group consisting of the lysine residue at position 10, the alanine residue at position 34, the alanine residue at position 47 and the arginine residue at position 59, wherein the test substance is judged to be capable of regulating the binding of telomeric DNA to TRF2 when the test substance interacted with the domain or the protein.
(13) The method of (12) above, wherein whether or not a TRF2 DNA-binding domain having an amino acid sequence as shown in SEQ ID NO: 2 or a protein comprising the domain interacts with a test substance at least at one amino acid site selected from the group consisting of the lysine residue at position 10, the alanine residue at position 34, the alanine residue at position 47 and the arginine residue at position 59 is analyzed in the presence of a duplex DNA comprising a sequence represented by 5'-TTAGGG-3'.

The term "TRF2" used herein refers to a protein consisting of 500 amino acids which specifically binds to a mammalian telomeric TTAGGG repeat sequence and is composed of an N-terminal basic region, a dimerization domain at the central part and a C-terminal DNA-binding domain.

The term "TRF2 DNA-binding domain" used herein refers to a region consisting of about 60 amino acids located at a C-terminal site in TRF2. Biding to DNA is possible with this region alone.

The term "mutant protein" used herein means a protein which is different from a standard protein but retains the essential nature of the standard protein. A typical mutant protein has an amino acid sequence which is different from the amino acid sequence of the standard protein.

5'-TTAGGG-3' is a repeat sequence in the duplex DNA of mammalian telomeres. Specific proteins (e.g., Rap1 in budding yeast, Tazlp in fission yeast, RTBP1 in rice, and TRF1 and TRF2 found in mammals) bind to this sequence.

As a specific example of duplex DNA comprising a sequence represented by 5'-TTAGGG-3', a DNA having the following sequences may be given. tr13; 5'-GTTAGGGTTAGGG-3' (SEQ ID NO: 17)/5'-CCCTAACCCTAAC-3' (SEQ ID NO: 18)

The ability of TRF2 DNA-binding domain or a mutant protein thereof to bind to duplex DNA comprising a sequence represented by 5'-TTAGGG-3' may be determined by surface plasmon resonance analyses as described later in Examples.

Chromosome ends in vertebrates have a region called telomeres composed of G-rich repetitive DNA sequence and a variety of proteins associated with the DNA. The term "telomeric DNA" used herein refers to this repetitive DNA sequence.

The term "antibody" used herein means a protein which is induced in the body by immunological reaction as a result of stimulation with antigen, and has activity of specifically binding to immunogen (antigen). This term encompasses polyclonal antibodies, monoclonal antibodies, chimeric antibodies, single chain antibody, humanized antibodies, and Fab or Fab fragments.

The term "interaction" used herein means a process by which two or more objects (e.g., atoms or molecules) have an effect on each other. Specific examples of interactions include, but are not limited to, hydrophilic interactions (e.g., hydrogen bonds or salt bridges), hydrophobic interactions (e.g., hydrophobic bonds), electrostatic interactions, and van der Waals interactions.

According to the present invention, a TRF2 mutant which has a much stronger binding ability to telomeric duplex DNA than a wild-type TRF2 has been provided.

Further, according to the present invention, the structure and function of complexes of telomeric protein TRF2 DNA-binding domain and telomeric duplex DNA have been analyzed. Based on the results, it has become possible to screen for drugs capable of regulating the binding ability of TRF2 to DNA.

The present specification encompasses the contents disclosed in the specification and/or the drawings of Japanese Patent Application No. 2004-46238 based on which the present application claims priority.

### Brief Description of Drawings

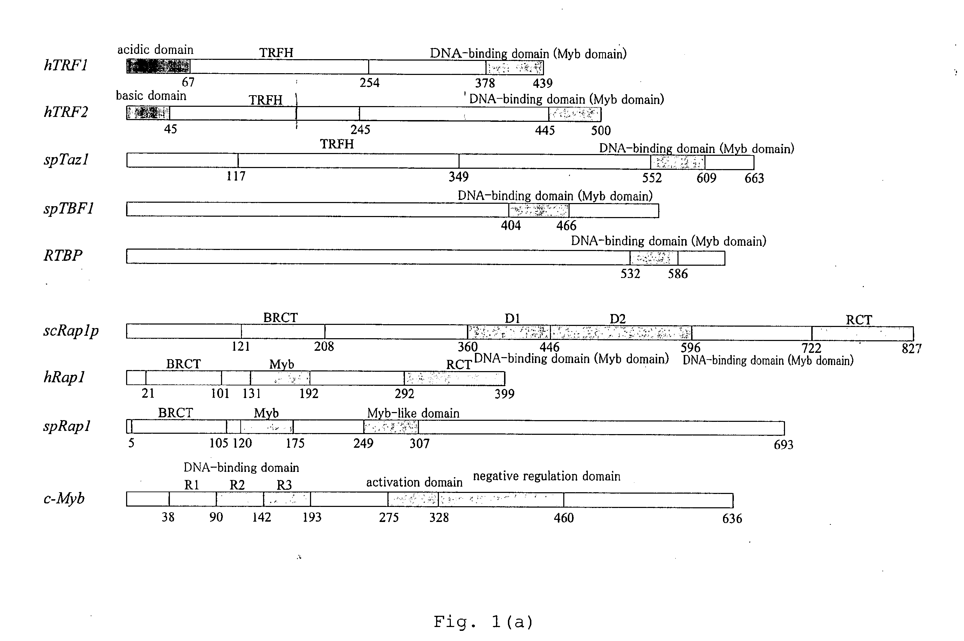

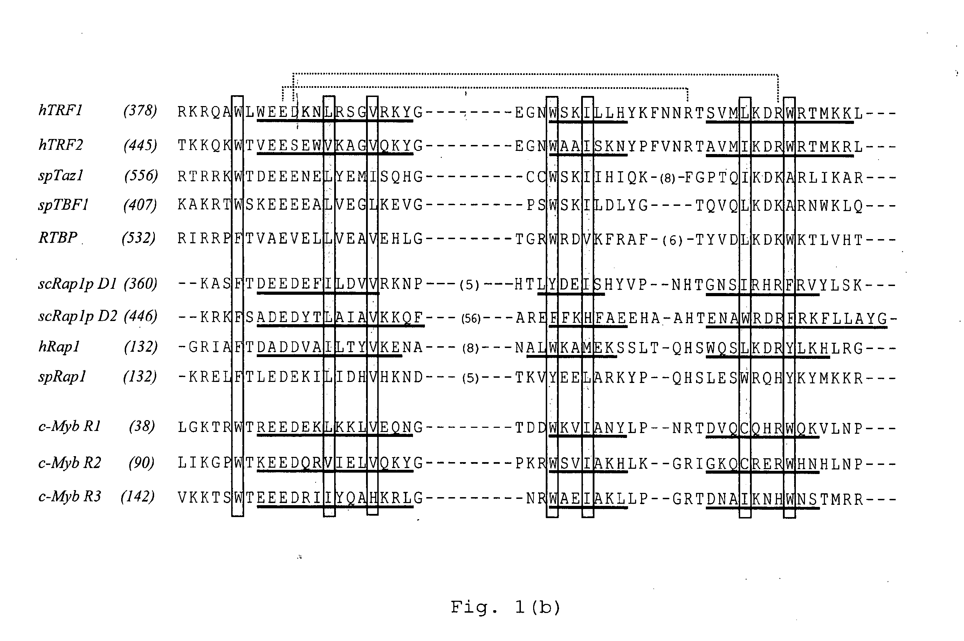

Fig. 1 (a) shows domain structures of Myb domain-containing major telomeric proteins and c-Myb. Basic: basic domain; Acidic: acidic domain; TRFH: TRF homology domain; BRCT: BRCA1 homology domain; RCT: Rap1 C-terminal homology domain; Active: transcription activation domain; Negative regulation: transcription negative regulation domain; Myb: Myb homology domain; DNA binding: Myb region with DNA binding ability.
Fig. 1 (b) shows amino acid sequences for the Myb domains of major telomeric proteins and the individual repeats (R1, R2 and R3) of c-Myb. Helical regions are underlined. Two pairs of polar residues that form an intramolecular salt bridge are connected with dotted lines. Dotted lines are connecting residues forming intramolecular salt bridges.
Fig. 2 (a) shows a stereo view of the DNA-binding domain (DBD) of TRF2. The upper panel shows the lowest energy structure in the 25 structures determined by NMR. The lower panel shows a superposition of the 25 structures.
Fig. 2 (b) shows a stereo view of a complex composed of TRF2-DBD and DNA. Panel (a) shows the lowest energy structure in the 20 structures determined by NMR. Panel (b) shows a superposition of the 20 structures.
Fig. 3 (a) a schematic diagram showing recognition of DNA by TRF1 and TRF2 based on their structures determined by NMR. Arrows with solid lines indicate hydrophilic interactions and arrows with dotted lines indicate hydrophobic interactions.
Fig. 3 (b) shows electrostatic potential surfaces of TRF1 and TRF2. Blue color represents positively charged area and red color represents negatively charged area. The indicated amino acid residues are involved in DNA recognition. The amino acid residues highlighted in red differ between TRF1 and TRF2.

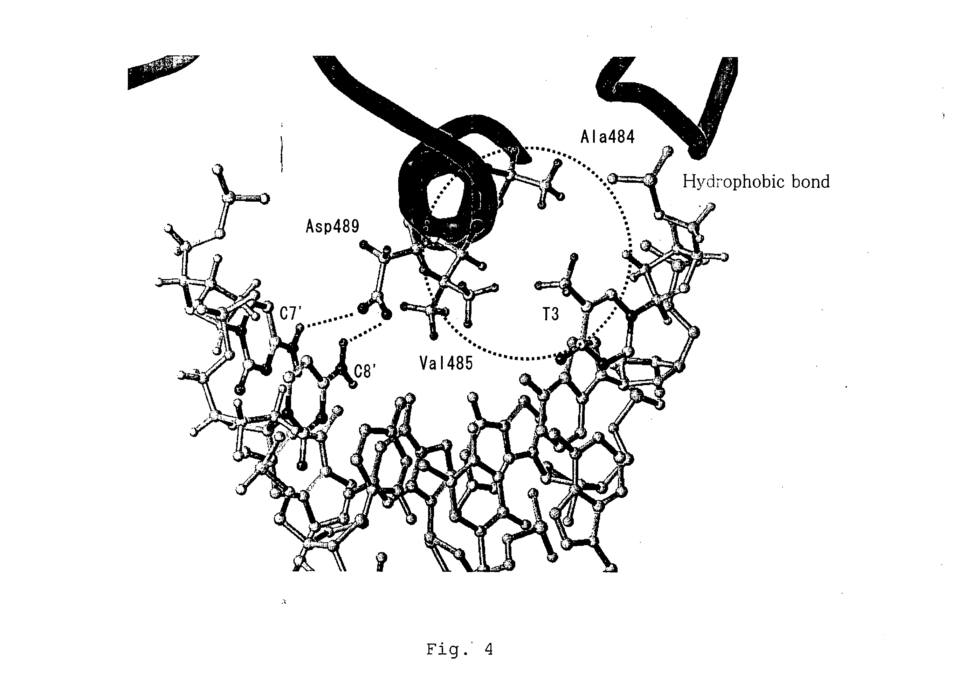

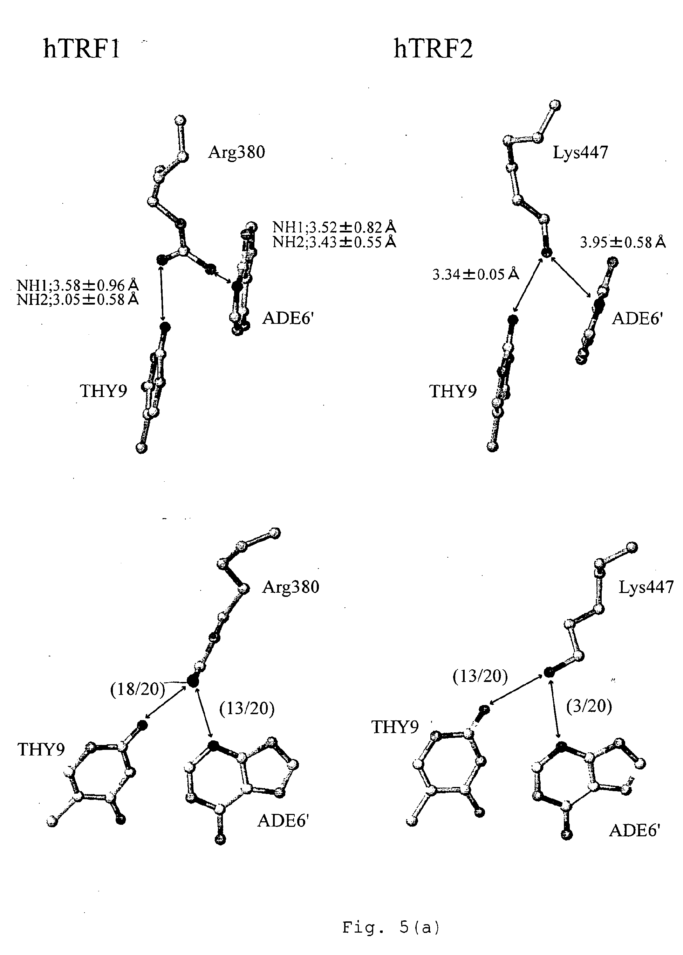

Fig. 4 shows the recognition of the major groove of DNA by TRF2. The dotted circle indicates hydrophobic interactions formed by T3, Va1485 and Ala484. The dotted lines connecting Asp489 to C7' and C8' indicate hydrogen bonds.
Fig. 5 (a) is a schematic drawing showing the recognition of the minor groove of DNA by TRF1 and TRF2. Numeric figures indicated in the upper panel show the distances between N1 and N2 of Arg380 and 02 of T9 and N3 of A6' (angstrom), and the distances between Lys447 and 02 of T9 and N3 of A6' (angstrom). Numeric figures in parentheses in the lower panel show the number of hydrogen bonds in the determined 20 structures which satisfy the following criteria: N-H··D (O or N): H··D distance < 2.7 Å; N··D distance<3.4 Å; N-H-D angle> 90°.
Fig. 5 (b) shows the interaction modes of Ser404/Ala471, Ser417/Ala484 and the phosphate skeleton of T3 in TRF1 and TRF2. Lines indicate hydrogen bonds.
Fig. 6 (a) shows imino proton signals of DNA when the molar ratio of individual DBDs of TRF2 and tr13 (5'-d(GTTAGGGTTAGGG)) is 1:1. Individual imino protons are indicated with the signals. Remarkably changed T8 and T9 are marked with a circle.
Fig. 6 (b) shows differences of the imino proton chemical shift values in mutants obtained in Fig. 6 (a) from the corresponding values of the wild type.

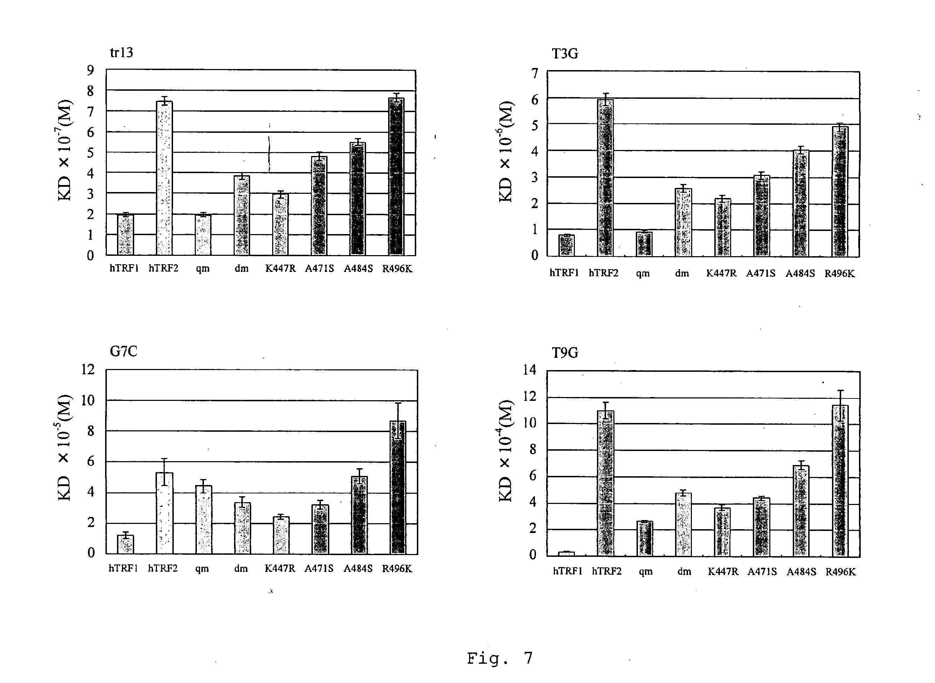

Fig. 7 shows the dissociation constants (KD) of individual DBDs of TRF2 obtained by SPR for tr13, T3G, G7C and T9G.

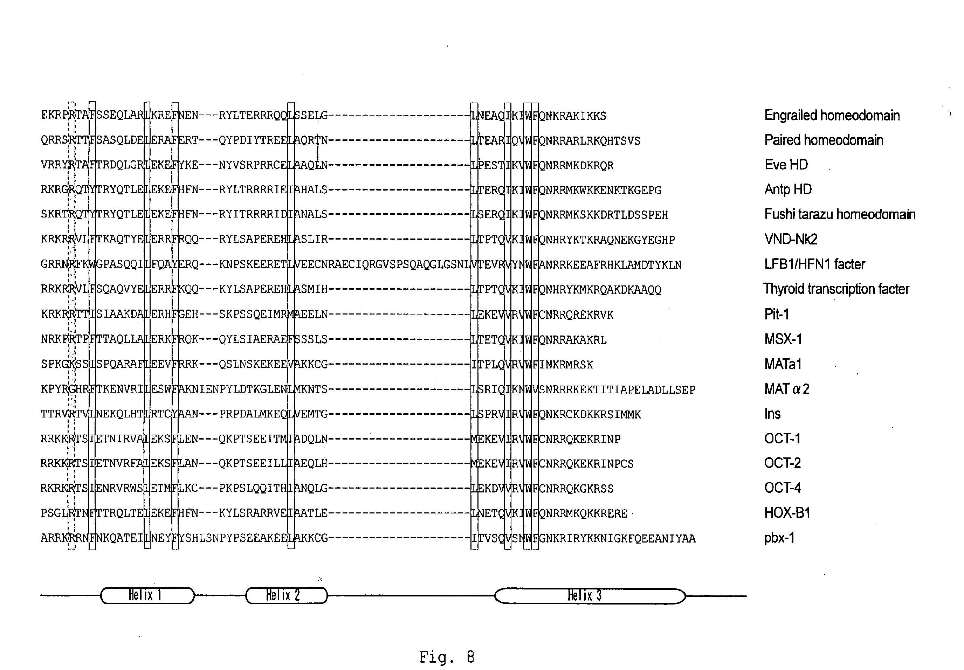

Fig. 8 shows an alignment of amino acid sequences for homeodomains whose structures are deposited. Conserved hydrophobic amino acids are colored in yellow (boxed with solid line) and the 5th amino acids are colored in cyan (boxed with dotted line).

### Best Mode for Carrying out the Invention

### 1. TRF2 DNA-Binging Domain Mutant Protein

The present invention provides a TRF2 DNA-binging domain mutant protein as shown in the following (a) or (b), or a salt thereof.
(a) a TRF2 DNA-binding domain mutant protein having an amino acid sequence as shown in SEQ ID NO: 2 but with at least one substitution selected from the group consisting of substitution of the lysine residue with arginine at position 10, substitution of the alanine residue with serine at position 34, substitution of the alanine residue with serine at position 47 and substitution of the arginine residue with lysine at position 59;
(b) a TRF2 DNA-binding domain mutant protein having an amino acid sequence of the mutant protein of (a) above but with one or several amino acid residues other than the amino acid residues at positions 10, 34, 47 and 59 being deleted, substituted or added, and which has a higher binding ability to a duplex DNA comprising a sequence represented by 5'-TTAGGG-3' than a wild-type TRF2 DNA-binding domain protein having an amino acid sequence as shown in SEQ ID NO: 2.

Specific examples of the TRF2 DNA-binding domain mutant protein of (a) above include, but are not limited to, the proteins of the following (ia) to (viiia).
(ia) a protein having an amino acid sequence as shown in SEQ ID NO: 2 but with the lysine residue at position 10 being substituted with arginine;
(iia) a protein having an amino acid sequence as shown in SEQ ID NO: 2 but with the alanine residue at position 34 being substituted with serine;
(iiia) a protein having an amino acid sequence as shown in SEQ ID NO: 2 but with the alanine residue at position 47 being substituted with serine;
(iva) a protein having an amino acid sequence as shown in SEQ ID NO: 2 but with the arginine residue at position 59 being substituted with lysine;
(va) a protein having an amino acid sequence as shown in SEQ ID NO: 2 but with the lysine residue at position 10 being substituted with arginine, the alanine residue at position 34 being substituted with serine, the alanine residue at position 47 being substituted with serine and the arginine residue at position 59 being substituted with lysine;
(via) a protein having an amino acid sequence as shown in SEQ ID NO: 2 but with the lysine residue at position 10 being substituted with arginine and the alanine residue at position 47 being substituted with serine;
(viia) a protein having an amino acid sequence as shown in SEQ ID NO: 2 but with the alanine residue at position 34 being substituted with serine and the alanine residue at position 47 being substituted with serine;
(viiia) a protein having an amino acid sequence as shown in SEQ ID NO: 2 but with the lysine residue at position 10 being substituted with arginine, the alanine residue at position 34 being substituted with serine and the alanine residue at position 47 being substituted with serine.

The amino acid sequence for the protein of (ia) above is shown in SEQ ID NO: 4.

The amino acid sequence for the protein of (iia) above is shown in SEQ ID NO: 6.

The amino acid sequence for the protein of (iiia) above is shown in SEQ ID NO:8.

The amino acid sequence for the protein of (iva) above is shown in SEQ ID NO: 10.

The amino acid sequence for the protein of (va) above is shown in SEQ ID NO: 12.

The amino acid sequence for the protein of (viia) above is shown in SEQ ID NO: 14.

Specific examples of the TRF2 DNA-binding domain mutant protein of (b) above include, but are not limited to, mutants of wild-type TRF2 DNA-binding domain derived from organisms other than human (e.g., yeast, rice, vertebrates, etc.) and proteins having an amino acid sequence as shown in SEQ ID NO: 4, 6, 8, 10, 12 or 14 with a methionine residue added at the N-terminus (i.e., at position 1).

The TRF2 DNA-binding domain mutant protein of the invention may be either a mutant of human-derived wild-type TRF2 DNA-binding domain or a mutant of wild-type TRF2 DNA-binding domain derived from organisms other than human (e.g., yeast, rice, vertebrates, etc.) .

The TRF2 DNA-binding domain mutant protein or a salt thereof of the invention may be prepared by known methods. For example, a DNA encoding a TRF2 DNA-binding domain mutant protein may be obtained as described later in sub-section 2. The resultant DNA may be integrated into an appropriate vector, introduced into an appropriate host, and then expressed as a recombinant protein. Thus, the TRF2 DNA-binding domain mutant protein can be produced (see, for example, Current Protocols Compact Version: Molecular Biology Experimental Protocols I, II and III translated by Kaoru Saigo and Yumiko Sano, published by Maruzen; and its original version, Ausubel et al., Short Protocols in Molecular Biology, Third Edition, John Wiley & Sons, Inc., New York).

Alternatively, the TRF2 DNA-binding domain mutant protein or a salt thereof of the invention may be prepared by a known peptide synthesis method.

The TRF2 DNA-binding domain mutant protein of the invention may be obtained in a salt form by a known method. The salt of the TRF2 DNA-binding domain mutant protein of the invention may be a pharmacologically acceptable salt. Particularly preferable are pharmacologically acceptable acid addition salts. Examples of such acid addition salts include salts formed with inorganic acids (e.g. hydrochloric acid, phosphoric acid, hydrobromic acid or sulfuric acid) and salts formed with organic acids (e.g. acetic acid, formic acid, propionic acid, fumaric acid, maleic acid, succinic acid, tartaric acid, citric acid, malic acid, oxalic acid, benzoic acid, methanesulfonic acid or benzenesulfonic acid).

### 2. Isolated DNA Encoding TRF2 DNA-Binding Domain Mutant Protein

An isolated DNA encoding the TRF2 DNA-binding domain mutant protein of the invention may be any DNA as long as it comprises a nucleotide sequence encoding the TRF2 DNA-binding domain mutant protein of the invention. As a specific example of the DNA encoding the TRF2 DNA-binding domain mutant protein of the invention, a DNA comprising the nucleotide sequence as shown in SEQ ID NO: 3, 5, 7, 9, 11 or 13 may be given.

The isolated DNA encoding the TRF2 DNA-binding domain mutant protein of the invention may be prepared, for example, as described below.

Briefly, mRNA is extracted from human cells and cDNA is synthesized from the mRNA using a reverse transcriptase and oligo dT primers. Then, the coding region of TRF2 DNA-binding domain (63 residues) is amplified by PCR. The resultant PCR product is a DNA encoding a region containing a wild-type hTRF2 DNA-binding domain. One example of an amino acid sequence of a region containing a wild-type hTRF2 DNA-binding domain and one example of a nucleotide sequence encoding the domain are shown in SEQ ID NO: 2 and SEQ ID NO: 1, respectively.

The DNA encoding the hTRF2 DNA-binding domain mutant protein of the invention may be prepared by mutating the coding region of hTRF2 DNA-binding domain (63 residues) by point mutation mutagenesis. The mutated coding region of hTRF2 DNA-binding domain (63 residues) is amplified by PCR. The resultant PCR product is a DNA encoding a hTRF2 DNA-binding domain mutant protein. One example of the nucleotide sequence of a DNA encoding a protein having an amino acid sequence as shown in SEQ ID NO: 2 but with the lysine residue at position 10 being substituted with arginine is shown in SEQ ID NO: 3. One example of the nucleotide sequence of a DNA encoding a protein having an amino acid sequence as shown in SEQ ID NO: 2 but with the alanine residue at position 47 being substituted with serine is shown in SEQ ID NO: 7. One example of the nucleotide sequence of a DNA encoding a protein having an amino acid sequence as shown in SEQ ID NO: 2 but with the arginine residue at position 59 being substituted with lysine is shown in SEQ ID NO: 9. One example of the nucleotide sequence of a DNA encoding a protein having an amino acid sequence as shown in SEQ ID NO: 2 but with the lysine residue at position 10 being substituted with arginine, the alanine residue at position 34 being substituted with serine, the alanine residue at position 47 being substituted with serine and the arginine residue at position 59 being substituted with lysine is shown in SEQ ID NO: 11. One example of the nucleotide sequence of a DNA encoding a protein having an amino acid sequence as shown in SEQ ID NO: 2 but with the lysine residue at position 10 being substituted with arginine and the alanine residue at position 47 being substituted with serine is shown in SEQ ID NO: 13.

### 3. Recombinant Vector

A recombinant vector comprising a DNA encoding the TRF2 DNA-binding domain mutant protein of the invention may be obtained by inserting a DNA encoding the TRF2 DNA-binding domain mutant protein of the invention into an appropriate expression vector according to known methods (e.g. methods described in Molecular Cloning 2nd Edition, J. Sambrook et al., Cold Spring Harbor Lab. Press, 1989).

Examples of expression vectors useful in the invention include, but are not limited to, plasmids derived from *Escherichia coli* (e.g. pBR322, pBR325, pUC12, and pUC13); plasmids derived from *Bacillus subtilis* (e.g. pUB110, pTP5 and pC194); plasmids derived from yeast (e.g. pSH19 and pSH15); bacteriophages such as λ-phage; animal viruses such as retrovirus, vaccinia virus; and insect pathogenic virus such as baculovirus.

The expression vector may comprise a promoter, enhancer, splicing signal, polyadenylation signal, selective markers, SV40 replication origin and the like.

The expression vector may be a fusion protein expression vector. Various fusion protein expression vectors are commercialized, e.g. pGEX series (Amersham Pharmacia Biotech), pET CBD Fusion System 34b-38b (Novagen), pET Dsb Fusion Systems 39b and 40b (Novagen), and pET GST Fusion System 41 and 42 (Novagen).

### 4. Transformant

A transformant may be obtained by introducing a recombinant vector comprising a DNA encoding the TRF2 DNA-binding domain mutant protein of the invention into a host.

Specific examples of hosts useful in the invention include, but are not limited to, bacterial cells (e.g. bacteria belonging to the genus *Escherichia,* bacteria belonging to the genus *Bacillus* such as *B. subtilis*), fungal cells (e.g. yeast, fungi belonging to the genus *Aspergillus),* insect cells (e.g. S2 cells, Sf cells), animal cells (e.g. CHO cells, COS cells, HeLa cells, C127 cells, 3T3 cells, BHK cells, HEK293 cells) and plant cells.

The introduction of a recombinant vector into a host may be performed by methods described in Molecular Cloning 2nd Edition, J. Sambrook et al., Cold Spring Harbor Lab. Press, 1989 (e.g. the calcium phosphate method, the DEAE-dextran method, transfection, microinjection, lipofection, electroporation, transduction, the scrape loading method, or the shot gun method) or infection.

The thus obtained transformant may be cultured in a medium, and a TRF2 DNA-binding domain mutant protein of interest may be recovered from the resultant culture. When the TRF2 DNA-binding domain mutant protein is secreted into the medium, the medium is recovered and then the TRF2 DNA-binding domain mutant protein is separated and purified therefrom. When the TRF2 DNA-binding domain mutant protein is produced within the transformed cell, the cell is lysed and then the TRF2 DNA-binding domain mutant protein is separated and purified from the resultant lysate.

When a TRF2 DNA-binding domain mutant protein of interest is expressed in the form of a fusion protein with other protein (which will function as a tag), first, the fusion protein is separated and purified. Then, the TRF2 DNA-binding domain mutant protein of interest can be obtained by cutting the other protein by treating the fusion protein with FactorXa or an appropriate enzyme (enterokinase).

The separation and purification of the TRF2 DNA-binding domain mutant protein may be performed by known methods. For example, methods utilizing the difference in solubility, such as salting out and solvent precipitation; methods utilizing difference in molecular weight, such as dialysis, ultra-filtration, gel filtration, and SDS-polyacrylamide gel electrophoresis; methods utilizing difference in electric charge, such as ion exchange chromatography; methods utilizing specific affinity, such as affinity chromatography; methods utilizing difference in hydrophobicity, such as reversed-phase high performance liquid chromatography; methods utilizing difference in isoelectric point, such as isoelectric focusing; and the like may be used.

### 5. Antibody

An antibody to the TRF2 DNA-binding domain mutant protein or a salt thereof of the invention may be used for detecting and/or quantitatively determining the TRF2 DNA-binding domain mutant protein or a salt thereof of the invention.

An antibody to the TRF2 DNA-binding domain mutant protein or a salt thereof of the invention may be obtained by administering to an animal the TRF2 DNA-binding domain mutant protein or a salt thereof of the invention or a fragment thereof containing the epitope of the protein, according to conventional protocols.

The antibody of the invention may be any one of polyclonal antibody, monoclonal antibody, chimeric antibody, single chain antibody or humanized antibody.

Polyclonal antibodies may be prepared according to known methods or modifications thereof. For example, a complex of an immunogen (antigen protein) and a carrier protein is prepared and then administered to animals (immunization). Fractions containing an antibody to the protein of the invention are harvested from the immunized animals, followed by separation and purification of the antibody. At the time of administration of the complex, complete Freund's adjuvant or incomplete Freund's adjuvant may also be administered to enhance antibody production ability. The administration is carried out generally once in about every 2-6 weeks and about 3-10 times in the total. Polyclonal antibodies can be recovered from the blood, abdominal dropsy or other body fluid, preferably from the blood, of the immunized animals. The separation and purification of polyclonal antibodies may be performed by the same methods for separation and purification of immunoglobulin (e.g. salting out, alcohol precipitation, isoelectric precipitation, electrophoresis, absorption and desorption with ion exchangers, ultracentrifugation, gel filtration, specific purification methods in which an antibody of interest alone is recovered using an antigen-bound solid phase or an active adsorbent such as protein A or protein G, followed by dissociation of the bond).

Monoclonal antibodies may be prepared by the hybridoma method of G. Koehler and C. Milstein described in Nature (1975) 256:495; Science (1980) 208:692. Briefly, after immunization of animals, antibody producing cells are isolated from the spleen of the immunized animals, and then fused to myeloma cells to thereby prepare monoclonal antibody-producing cells. Further, a cell line may be isolated therefrom which reacts specifically with the TRF2 DNA-binding domain mutant protein or a salt thereof but does not cross-react with other antigen proteins substantially. This cell line is cultured, and a monoclonal antibody of interest can be obtained from the resultant culture. Purification of monoclonal antibodies may be performed according to the above-described methods for separation and purification of immunoglobulin.

A method for preparing single chain antibodies is disclosed in U.S. Patent No. 4,946,778.

A method for preparing humanized antibodies is disclosed in Biotechnology 10:1121- (1992); Biotechnology 10:169- (1992).

### 6. Use of TRF2 DNA-Binding Domain Mutant Protein or a Salt Thereof

The TRF2 DNA-binding domain mutant protein or a salt thereof of the invention may be used for controlling events in which TRF2 is involved (e.g., cancer, senescence, apoptosis, etc.). Since it is known that senescence or apoptosis occurs when TRF2 loses its binding ability to DNA (Karlseder, J., Smogorzewska, A. & de Lange, T. Senescence induced by altered telomere state, not telomere loss. Science 295, 2446-2449. (2002); Smogorzewska, A. & de Lange, T. Different telomere damage signaling pathways in human and mouse cells, EMBO J. 21, 4338-4348. (2002); Kalseder, J., Broccoli, D., Dai, Y., Hardy, S. & de Lange, T. p53- and ATM-dependent apoptosis induced by telomeres lacking TRF2. Science 283, 1321-1325. (1999)), it is believed that senescence or apoptosis can be prevented by using the TRF2 DNA-binding domain mutant protein or a salt thereof of the invention.

When the TRF2 DNA-binding domain mutant protein or a salt thereof of the invention is administered to a subject, the TRF2 DNA-binding domain mutant protein or a salt thereof may be administered alone or together with carriers, diluents or excipients in an appropriate form of a pharmaceutical composition, orally or parenterally to mammals (e.g. human, rabbit, dog, cat, rat, mouse). Administration may be continued until efficacy of treatment is observed or until amelioration of symptoms is achieved at an appropriate dose, by an appropriate administration method and at appropriate frequency, depending on the severity of conditions or the response of the patient body.

The TRF2 DNA-binding domain mutant protein or a salt thereof of the invention may be used as a pharmaceutical product for preventing and/or treating diseases or as an experimental reagent.

### 7. Protein Comprising TRF2 DNA-Binding Domain Mutant Protein

The present invention also provides a protein comprising the above-described TRF2 DNA-binding domain mutant protein, and a salt thereof. As an example of the protein comprising the TRF2 DNA-binding domain mutant protein, a wild-type TRF2 wherein the DNA-binding domain is replaced with the TRF2 DNA-binding domain mutant protein of the invention may be given. The nucleotide sequence and amino acid sequence for wild-type human TRF2 are shown in SEQ ID NOS: 15 and 16. In the amino acid sequence as shown NO: 16, residues from positions 1 to 45 represent an N-terminal basic domain; residues from positions 46 to 245 represent a central, TRF specific/dimerization domain; and residues from positions 438 to 500 represent the DNA-binding domain. The amino acid sequence as shown in SEQ ID NO: 2 represents a fragment of SEQ ID NO: 16 spanning from Glu438 to Asn500. The amino acid positions 10, 34, 47 and 59 in SEQ ID NO: 2 correspond to the positions 447, 471, 484 and 496 in SEQ ID NO: 16, respectively.

The protein of the invention comprising a TRF2 DNA-binding domain mutant protein, or a salt thereof may be prepared by known methods. For example, a DNA encoding a protein comprising a TRF2 DNA-binding domain mutant protein may be obtained, integrated into an appropriate vector, introduced into an appropriate host, and then expressed as a recombinant protein. Thus, the protein comprising a TRF2 DNA-binding domain mutant protein can be produced (see, for example, Current Protocols Compact Version: Molecular Biology Experimental Protocols I, II and III translated by Kaoru Saigo and Yumiko Sano, published by Maruzen; and its original version, Ausubel et al., Short Protocols in Molecular Biology, Third Edition, John Wiley & Sons, Inc., New York).

Alternatively, the protein of the invention comprising a TRF2 DNA-binding domain mutant protein, or a salt thereof may be prepared by a known peptide synthesis method.

The protein of the invention comprising a TRF2 DNA-binding domain mutant protein may be obtained in a salt form by a known method. The salt of the protein comprising a TRF2 DNA-binding domain mutant protein may be a pharmacologically acceptable salt. Particularly preferable are pharmacologically acceptable acid addition salts. Examples of such acid addition salts include salts formed with inorganic acids (e.g. hydrochloric acid, phosphoric acid, hydrobromic acid or sulfuric acid) and salts formed with organic acids (e.g. acetic acid, formic acid, propionic acid, fumaric acid, maleic acid, succinic acid, tartaric acid, citric acid, malic acid, oxalic acid, benzoic acid, methanesulfonic acid or benzenesulfonic acid).

The protein of the invention comprising a TRF2 DNA-binding domain mutant protein, or a salt thereof may be used for controlling events in which TRF2 is involved (e.g., cancer, senescence, apoptosis, etc.). Since it is known that senescence or apoptosis occurs when TRF2 loses its binding ability to DNA (Karlseder, J., Smogorzewska, A. & de Lange, T. Senescence induced by altered telomere state, not telomere loss. Science 295, 2446-2449. (2002); Smogorzewska, A. & de Lange, T. Different telomere damage signaling pathways in human and mouse cells, EMBO J. 21, 4338-4348. (2002); Kalseder, J., Broccoli, D., Dai, Y., Hardy, S. & de Lange, T. p53-and ATM-degendent apoptosis induced by telomeres lacking TRF2. Science 283, 1321-1325. (1999)), it is believed that senescence or apoptosis can be prevented by using the protein of the invention comprising a TRF2 DNA-binding domain mutant protein, or a salt thereof.

The method of administration of the protein is as described above.

The protein of the invention comprising a TRF2 DNA-binding domain mutant protein, or a salt thereof may be used as a pharmaceutical product for preventing and/or treating diseases or as an experimental reagent.

### 8. Telomeric DNA Mutant

The present invention provides a DNA having a nucleotide sequence as shown in SEQ ID NO: 17 but with at least one substitution selected from the group consisting of substitution of the T at position 3 with G, substitution of the G at position 7 to C and substitution of the T at position 9 to G (hereinafter, sometimes referred to as "telomeric DNA mutant").

Examples of the telomeric DNA mutant of the invention include, but are not limited to, the DNAs of the following (ib) to (iiib).
(ib) a DNA having a nucleotide sequence as shown in SEQ ID NO: 17 with the T at position 3 being substituted with G
(iib) a DNA having a nucleotide sequence as shown in SEQ ID NO: 17 with the G at position 7 being substituted with C
(iiib) a DNA having a nucleotide sequence as shown in SEQ ID NO: 17 with the T at position 9 being substituted with G

The nucleotide sequence of the DNA of (ib) is shown in SEQ ID NO: 19.

The nucleotide sequence of the DNA of (iib) is shown in SEQ ID NO: 20.

The nucleotide sequence of the DNA of (iiib) is shown in SEQ ID NO: 21.

The telomeric DNA mutant of the invention may be single-stranded; double-stranded; or partially single-stranded and partially double-stranded. However, the mutant is preferably double-stranded considering the binding to a protein specific to the repetitive sequence in telomeric double-stranded DNA (i.e., telomeric protein).

The telomeric DNA mutant of the invention may be synthesized by known methods. For example, the telomeric DNA mutant may be synthesized with a commercial DNA synthesizer.

The telomeric DNA mutant of the invention may be used for analyzing the function of telomeric proteins (such as TRF1 and TRF2), as described later in Examples. Further, it is believed that the telomeric DNA mutant of the invention may be used for controlling events (e.g., cancer, senescence, apoptosis, etc.) in which telomeric proteins (such as TRF1 and TRF2) are involved.

The telomeric DNA mutant of the invention may be used as a pharmaceutical product for preventing and/or treating diseases or as an experimental reagent.

### 9. Complex Composed of TRF2 DNA-Binding Domain Mutant Protein or a Protein Comprising the Mutant Protein and DNA

The present invention provides a complex composed of the above-described TRF2 DNA-binding domain mutant protein or the protein comprising the mutant protein and DNA. The protein which constitutes the complex of the invention is a TRF2 DNA-binding domain mutant protein of the following (a) or (b), or a protein comprising the TRF2 DNA-binding domain mutant protein.
(a) a TRF2 DNA-binding domain mutant protein having an amino acid sequence as shown in SEQ ID NO: 2 but with at least one substitution selected from the group consisting of substitution of the lysine residue with arginine at position 10, substitution of the alanine residue with serine at position 34, substitution of the alanine residue with serine at position 47 and substitution of the arginine residue with lysine at position 59.
(b) a TRF2 DNA-binding domain mutant protein having an amino acid sequence of the mutant protein of (a) above but with one or several amino acid residues other than the amino acid residues at positions 10, 34, 47 and 59 being deleted, substituted or added, and which has a higher binding ability to a duplex DNA comprising a sequence represented by 5'-TTAGGG-3' than a wild-type TRF2 DNA-binding domain protein having an amino acid sequence as shown in SEQ ID NO: 2.

Examples of the TRF2 DNA-binding domain mutant protein of (a) include a protein having the amino acid sequence as shown in SEQ ID NO: 4, 6, 8, 10, 12 or 14.

Examples of the protein comprising the TRF2 DNA-binding domain mutant protein of (a) include a wild-type TRF2 in which the DNA-binding domain is replaced with a protein having the amino acid sequence as shown in SEQ ID NO: 4, 6, 8, 10, 12 or 14. The amino acid sequence for wild-type human TRF2 is shown in SEQ ID NO: 16. In the amino acid sequence of SEQ ID NO: 16, a region spanning from Glu438 to Asn500 is the DNA-binding domain.

As an example of the DNA which constitutes the complex of the invention, a duplex DNA comprising a sequence represented by 5'-TTAGGG-3' may be given. Examples of the DNA comprising a sequence represented by 5'-TTAGGG-3' include a DNA having the following sequences.
tr13: 5'-GTTAGGGTTAGGG-3' (SEQ ID NO: 17)/ 5'-CCCTAACCCTAAC-3' (SEQ ID NO: 18)

The complex of the invention may be formed as described below.

Briefly, the DNA and the protein are mixed in a solution with a salt concentration of 50 mM or more at pH 6.0-8.0 at 30°C or below. In the mixing, the protein should be added to the DNA, and the molar ratio of protein should not exceed the molar ratio of DNA. When it is necessary to reduce the salt concentration, once a complex is formed under the conditions described above and then the solution is exchanged by dialysis.

Formation of complex may be confirmed by using a Biacore (Biamolecular Interaction Analysis Core technology) apparatus (Biacore). For example, the TRF2 DNA-binding domain mutant protein of the invention or a protein comprising the mutant protein may be immobilized on a sensor chip and then contacted with the DNA on this sensor chip. Alternatively, the DNA may be immobilized on a sensor chip and then contacted with the TRF2 DNA-binding domain mutant protein of the invention or a protein comprising the mutant protein on this sensor chip. Extremely small mass changes on the surface of the sensor chip caused by association/dissociation between two molecules are detected as SPR (Surface Plasmon Resonance) signals. The time course of these signals are displayed as a graph called sensorgram, which is then analyzed with a software to thereby obtain K_{D} values. When the resultant K_{D} value is 10⁻⁴ or less, preferably 10⁻⁵ or less, more preferably 10⁻⁶ or less, it can be said that the protein and the DNA are specifically bound, i.e., forming a complex.

### 10. Method of Screening

The present invention provides a method of screening for substances which are capable of regulating the binding of telomeric DNA to TRF2, comprising analyzing whether or not a TRF2 DNA-binding domain having an amino acid sequence as shown in SEQ ID NO: 2 or a protein comprising the domain interacts with a test substance at least at one amino acid site selected from the group consisting of the lysine residue at position 10, the alanine residue at position 34, the alanine residue at position 47 and the arginine residue at position 59, and judging that the test substance is capable of regulating the binding of telomeric DNA to TRF2 when the test substance interacted with the domain or the protein. In this method of the invention, whether or not a TRF2 DNA-binding domain having an amino acid sequence as shown in SEQ ID NO: 2 or a protein comprising said domain interacts with a test substance at least at one amino acid site selected from the group consisting of the lysine residue at position 10, the alanine residue at position 34, the alanine residue at position 47 and the arginine residue at position 59 may be analyzed in the presence of a duplex DNA comprising a sequence represented by 5'-TTAGGG-3' . Examples of the DNA comprising a sequence represented by 5'-TTAGGG-3' include a DNA having the following sequences.
tr13: 5'-GTTAGGGTTAGGG-3' (SEQ ID NO: 17)/ 5'-CCCTAACCCTAAC-3' (SEQ ID NO: 18)

For analyzing whether or not a TRF2 DNA-binding domain having an amino acid sequence as shown in SEQ ID NO: 2 or a protein comprising this domain interacts with a test substance at least at one amino acid site selected from the group consisting of the lysine residue at position 10, the alanine residue at position 34, the alanine residue at position 47 and the arginine residue at position 59, the following methods or judgments may be used: tertiary structure analyses such as X-ray crystallography, nuclear magnetic resonance (NMR), neutron diffraction; methods of observing the shape of complexes with an electron microscope or atomic force microscope; techniques such as docking study in which stable binding modes between the tertiary structure of TRF2 DNA-binding domain having the amino acid sequence as shown in SEQ ID NO: 2 and a test substance of any structure are simulated using a computer; and judgments based on similarity to the tertiary structure of human TRF2 disclosed herein by the present inventors.

Substances capable of controlling the binding of telomeric DNA and TRF2 may have physiological activities such as promoting or delaying the senescence of cells, delaying the oncogenic transformation of cells, or promoting the death of cancer cells. Therefore, those substances which are judged to be capable of controlling the binding of telomeric DNA and TRF2 by the screening method of the invention may be used as an anticancer agent, cell senescence inhibitor, cell senescence promoter, or the like.

### [EXAMPLES]

Hereinbelow, the present invention will be described more specifically with reference to the following Examples. These Examples are provided only for the purpose of explanation and they are not intended to limit the scope of the present invention.

### "Results and Discussion"

### Structure Determination

The structures of complexes composed of a region comprising hTRF2 DNA-binding domain (hTRF2DBD: Glu438 to Asn500 with Met added to the N-terminus) and DNA were determined by NMR. The protein of interest (hTRF2DBD) was produced in an *E. coli* high expression system and labeled with ¹⁵N or ¹⁵N/¹³C. The DNA used in the complex was tr13: 5'-GTTAGGGTTAGGG-3' which had been used in the structure determination of hTRF1-DNA complex reported previously. In DNA-free structures and complex structures, 910 and 1412 NOEs were classified into four ranges depending on intensity (1.8-3.0, 2.3-4.0, 2.3-5.0 and 2.3-6.0), respectively. The restraint angle ranges were -90° < ϕ < -40° for ³JHNα <5.5Hz and -160°<ϕ <-80° for ³JHNα > 8.5Hz. For DNA-free structures, 25 structures with the lowest energy were selected from those satisfying less than 0.3 Å for the distance constraints and less than 5° for the dihedral angle restraints and showing no structural contradiction. In the calculation for complexes, Watson-Crick base pairing was maintained in the DNA by the following hydrogen bond restraints: for GC base pair, r_{G(N1)-C(N3)} = 2.95 ± 0.2 Å, r_{G(N2)-C(O2)} = 2.86 ± 0.2 Å, and r_{G(O6)-C(N4)} = 2.91 ± 0.2 Å and for TA base pair, r_{A(N6)-T(O4)} = 2.95 ± 0.2 Å, and r_{A(N1)-T(N3)}= 2.82 ± 0.2 Å. Angle restraints for the DNA were (α = -65°± 50°, β = 180° ± 50°, γ = 60° ± 50°, ε = 180° ± 50°, and ζ = -85° ± 50°) within the range satisfying both A- and B-form DNA conformers. With the above-described conditions, structure calculations were carried out with CNS (crystallography and NMR system; Yale University). In the calculation for complexes, first, calculations were carried out using NOEs from hTRF2DBD alone to thereby determine 200 structures satisfying the conditions. Subsequently, typical B-form DNA was placed 50 Å away from these 200 structures in various orientations, and calculations were carried out using all the restraints. Finally, 20 structures were selected which satisfy 0.3 Å for the distance constraints and less than 5° for the dihedral angle restraints and show no structural contradiction.

### Structure of hTRF2 DNA-Binding Domain

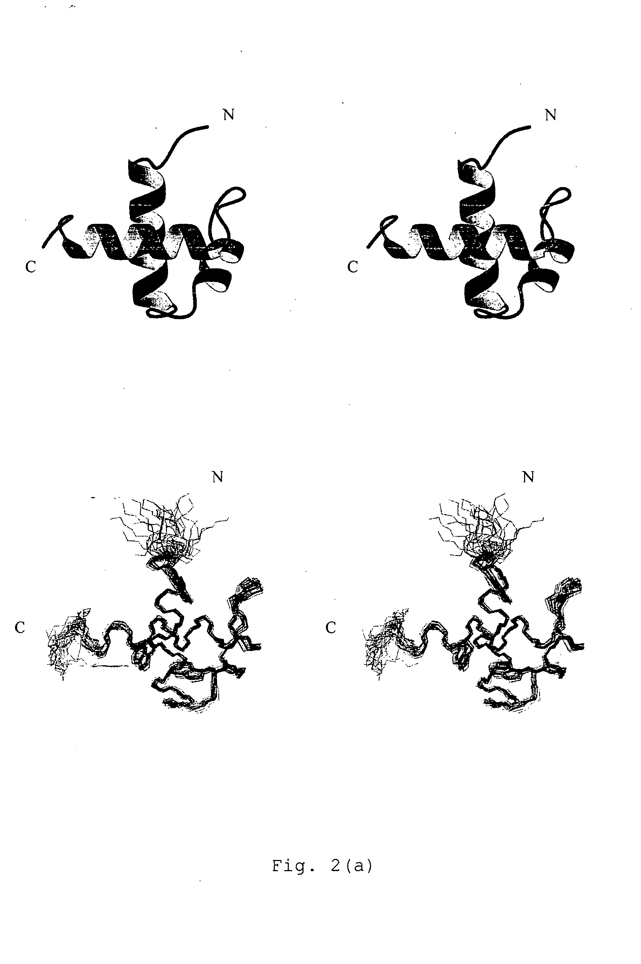

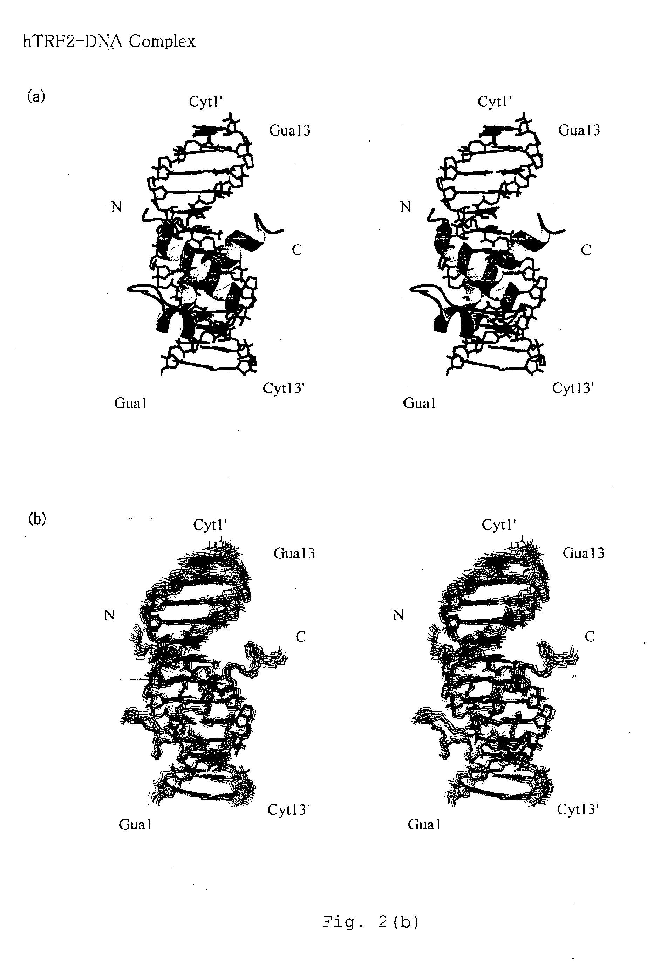

Fig. 2 (a) shows the determined 25 structures of hTRF2DBD (from Glu438 to Asn500 with Met added to the N-terminal) comprising hTRF2 DNA-binding domain. The overall root-mean square deviation (rmsd) values between the 25 individual structures and an averaged structure were 0.34 ± 0.05 Å for backbone atoms and 0.82 ± 0.06 Å for all heavy atoms. The basic structure of hTRF2 DNA-binding domain was almost identical to that of hTRF1 with some difference found in interhelix loops. Trp450, Val458, Leu462, Trp470, Leu474, Phe479, Ile487, Trp491 and Met494 formed a hydrophobic core to stabilize the structure of the protein. Further, salt bridges formed by Glu453/Arg482 and Ser454/Arg490, respectively, also contributed to the stabilization of the structure. This structure is a typical Myb structure known to date.

### Structure of hTRF2DBD-DNA Complex

The DNA used for the structural analysis of complex was tr13: 5'-GTTAGGGTTAGGG-3' which had been used in the structure determination of hTRF1-DNA complex reported previously. First, in order to examine whether hTRF2 is capable of binding to tr13 like TRF1, NMR titration experiments were carried out. As a result of adding the protein to the DNA, it was found that hTRF2DBD binds to the DNA at 1:1 as seen in hTRF1. Then, samples were prepared to give a DNA:protein ratio of 1:1. The structures of 20 complexes were determined by NMR (Fig. 2 (b)). In the structure of hTRF2DBD-tr13 complex, the rmsd values between individual structures and_an averaged structure were 0.51 ± 0.11 Å for backbone atoms and 0.68 ± 0.09 Å for all heavy atoms. The protein structure within the complex did not show any structural changes compared to DNA-free structures, and the DNA within the complex did not show any changes such as bending.

### DNA Recognition by the Complex

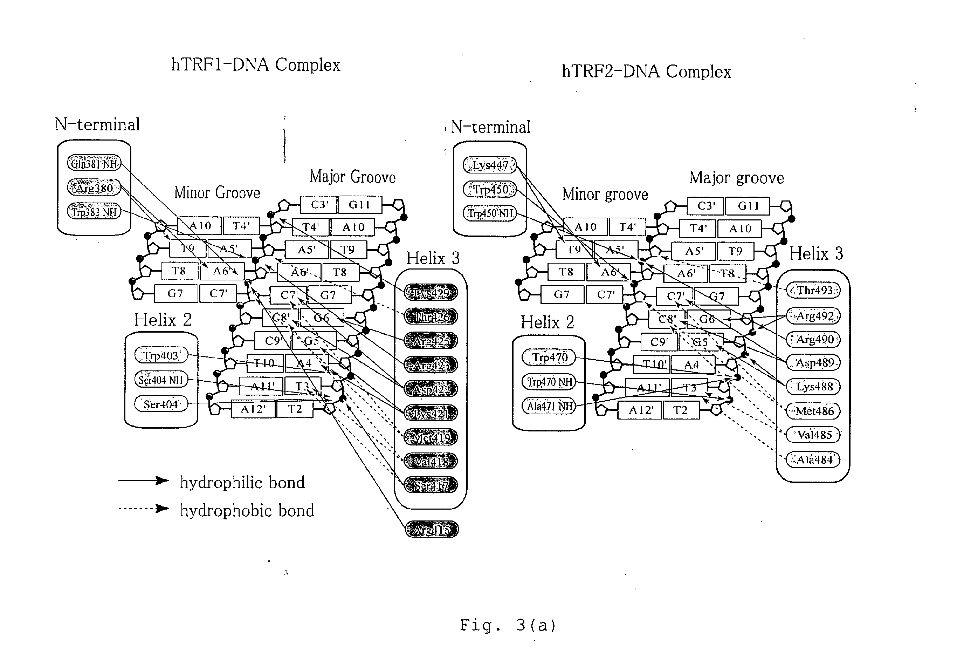

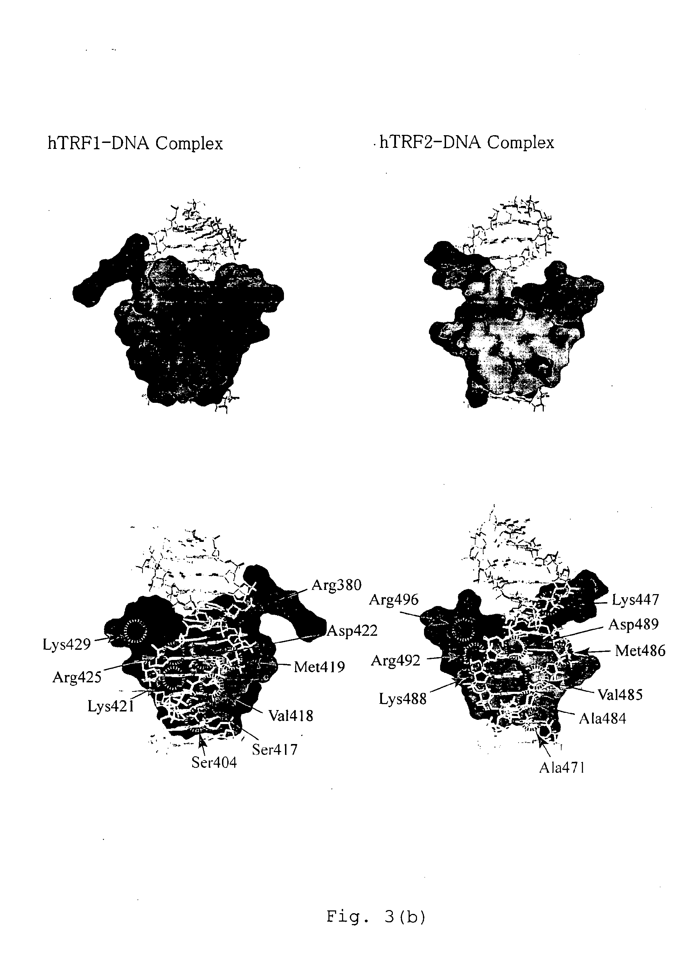

DNA recognition by hTRF2DBD in complex is shown in a schematic diagram in Fig. 3 (a). hTRF2DBD specifically recognizes DNA sequence by placing the helix3 in the major groove; Asp489 recognizes C7' and C8' and Lys488 recognizes A4 or G5. The methyl groups of Ala484 and Val485 make hydrophobic contacts with the methyl group of T3. Further, the methyl groups of Met486 and Val485 and the methylene group of Asp489 make hydrophobic contacts with bases of C7' and C8' and the backbone sugar of C7'. It was also confirmed that the methyl group of Thr493 makes hydrophobic contact with the sugar chain of A5'. In the minor groove, Lys447 recognizes T9 or A6'. In addition to direct recognition of bases, Trp450, Trp470, Ala471, Lys488, Arg490 and Arg492 recognize the phosphate skeleton of DNA to thereby enable interaction with the DNA. Such a mechanism of DNA recognition is observed in complexes of homeodomains reported to date and hTRF1 complexes. In particular, hTRF1 and hTRF2 recognize similar DNA sequence and have almost identical DNA-binding domain structures. It was confirmed that the DNA-binding modes are identical in hTRF1 and hTRF2 (Fig. 3 (b)).

### Difference in DNA Recognition between hTRF1 and hTRF2

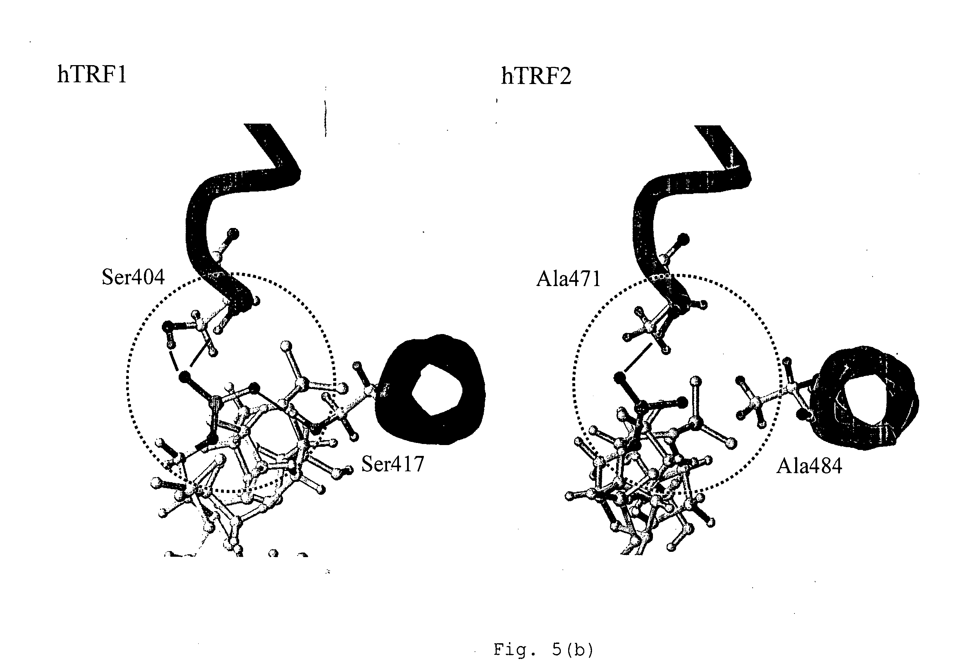

The DNA binding domain of hTRF2 (Thr445-Leu497) shows approximately 59% sequence identity and approximately 70% similarity to the corresponding region of hTRF1 (Arg378-Leu430). From the determined hTRF1 complexes and hTRF2 complexes, it was found that the differences in amino acids involved in DNA recognition are only four residues; they are Lys447, Ala471, Ala484, and Arg496 in hTRF2. However, the differences in these four amino acid residues do not cause changes in the major DNA recognition mode of hTRF2. DNA recognition of amino acids other than the above-described four in hTRF2 was completely identical to that of hTRF1. The corresponding four residues are Arg380, Ser404, Ser417, and Lys429 in hTRF1. In TRF2, Lys447 contacts T9, but in hTRF1, Arg380 contacts T9 and A6' also in the minor groove. In hTRF2, the NH of Ala471 main chain contacts the phosphate group of T3, and the methyl group of Ala484 side chain makes hydrophobic contact with the methyl group of T3. In hTRF1, these two residues are substituted with Ser. The main chain of Ser404 corresponding to Ala471 in hTRF1 makes the same contact, but its side chain also contacts the phosphate group. In Ser417 also, hydrophobic interaction and contact with the phosphate group as seen in Ala484 were confirmed (Fig. 5). In hTRF2, Arg496 is not involved in contact with DNA. In hTRF1, although Lys429 contacts with the phosphate group of T4', it recognizes only half or less of the structure. Therefore, it is believed that this residue makes little contribution to DNA recognition. Because of the difference in the four amino acids, hTRF2 complex has a smaller number of hydrogen bonds than hTRF1 complex. Thus, it is expected that hTRF2 would bind to tr13mer DNA with lower affinity than hTRF1.

### Analysis of Affinity to DNA

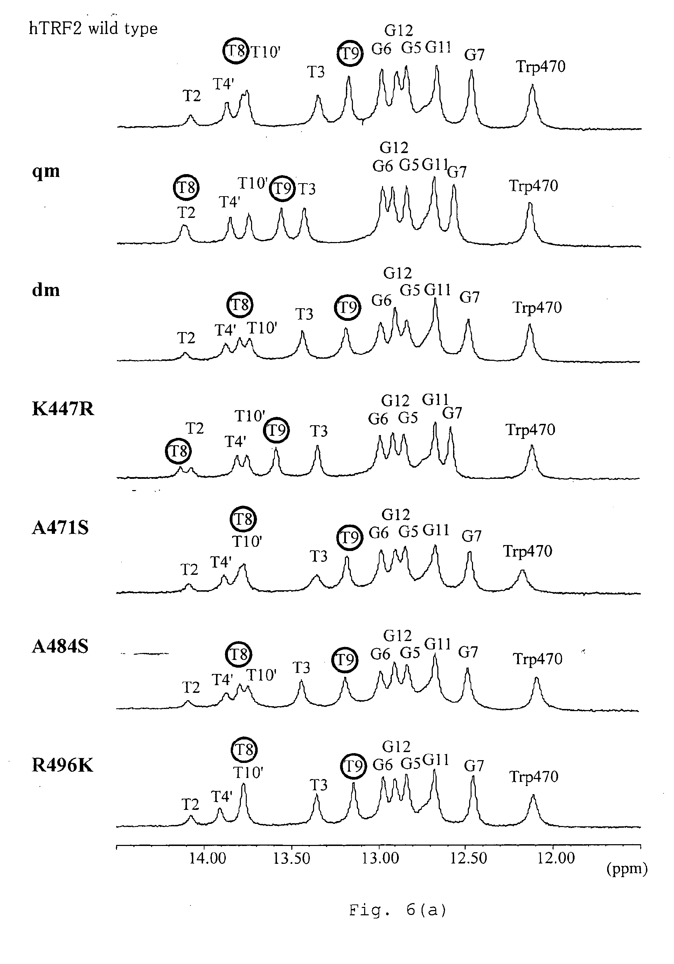

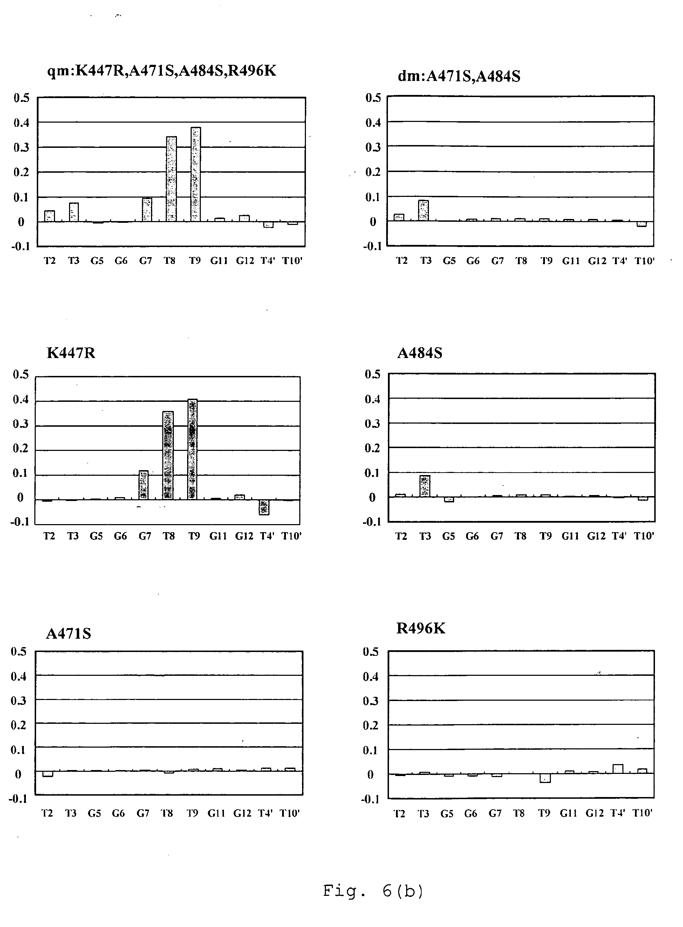

In order to examine the contributions of the four amino acids described above to the DNA-binding activities, the inventors created six mutants of hTRF2 in which the four critical amino acids are changed to the corresponding amino acids in of hTRF1, to thereby examine differences from wild-type hTRF2. The six mutants included four single mutants (K447R, A471S, A484S and R496K), one quadruple mutant designated qm (K447R/A471S/A484S/R496K), and one double mutant designated dm (A471S/A484S). First, NMR titration experiments were performed on these six mutants with tr13 DNA. The spectra of imino protons and the chemical shift changes of each mutant from the wild type are summarized in Figure 6. Fig. 6 (a) shows the spectra of individual imino protons. T8 and T9 which showed great changes are marked with circles. In K447R, big chemical shift changes were observed for the imino protons G7, T8 and T9. This suggests that as a result of substitution of Lys447 with Arg, the substituted Arg residue in the mutant interacts not only with T9 but also with A6'. In order to examine how this difference affects the affinity to DNA, affinity experiments were performed by surface plasmon resonance (SPR).

The result of SPR analysis of wild-type hTRF2 indicated an equilibrium dissociation constant (Kd) value of (7.48±0.21) x 10⁻⁷M for tr13 5'-GTTAGGGTTAGGG-3'), under conditions of 10 mM HEPES-KOH pH 6.8, 3 mM EDTA, 180 mM KCl, 0.003% (v/v) X-100. Subsequently, the same experiments were performed on three DNAs in which the base involved in specific recognition was changed: T3→G3 (TG3), G7→C7 (G7C) and T9→G9 (T9G). The results were as follows: T3G: Kd=(5.94±0.24) x 10⁻⁶M; G7C: Kd=(5.33±0.88) x 10⁻⁵M; and T9G: Kd=(1.10±0.06) x 10⁻⁴M (Table 2). Since the Kd value for T9G is more than 100 times larger than that for tr13, it is understood that T9 is a very important base. The same experiments were performed on the six mutants of hTRF2 under the same conditions. As a result, the mutants other than R496K showed increased affinity. In particular, qm with four mutations has about 4 times larger affinity (Kd=(1.96±0.09) x 10⁻⁷M than the wild-type. For the single mutants of hTRF2, Kd values were obtained as follows: K447R: Kd=(2.97±0.18) x 10⁻⁷M; A471 S: Kd=(4.82±0.20) x 10⁻⁷M; A484S: Kd=(5.50±0.17) x 10⁻⁷M; and R496K: Kd=(7.64±0.21) x 10⁻⁷M. It is noted that the affinity of K447R which recognizes T9 is higher than other those of other mutants. From these results, it is understood that in the minor groove, recognition of N-terminal arm greatly contributes to the affinity of DNA-binding domain to DNA and that hTRF2 has lower affinity to DNA than hTRF1 because hTRF2 recognizes DNA with Lys. Further, the six mutants were analyzed by SPR for the three DNAs T3G, G7C and T9G, as well as tr13. As a result, Kd values for T3G are about 7 times higher than those values for tr13; and Kd values for T9G are about 100 times higher than those values for tr13. However, Kd values for G7C showed different tendency of affinity. For example, K447R showed a lower Kd value than qm. Since C7' is changed to G7' in G7C, Asp489 which recognizes C7' has become incapable of recognizing DNA. Thus, interaction with DNA is assumed to be non-specific. Although the affinity to T9G is about 10 times lower than the affinity to G7C, differences in Kd values of individual mutants for DNA show relatively similar tendencies to those observed in tr13 and T3G. It is believed that the binding mode between the major groove and recognition helix is not changed and that only the affinity to T9G is merely very low. These results also indicate that the affinity to DNA is highly affected by the recognition of N-terminal arm in the minor groove.

The same experiments were performed on hTRF1 under the same conditions. As a result, the Kd value of hTRF1 for tr13 was (1.86±0.06) x 10⁻⁷M which was almost equal to the corresponding value shown by qm.

### Comparison with Homeodomains

Like Myb domain, homeodomains are composed of three helices. The second and third helices form a helix turn helix (HTH) motif and the third helix recognizes DNA in the major groove. Generally, the length of DNA sequence that a DNA binding motif can recognize is about 4-5 base pairs. Therefore, a plurality of subdomains each with a DNA recognition motif exist in c-Myb or scRaplp and they recognize a DNA cooperatively. Alternatively, intermolecular formation of homodimers or heterodimers makes the length of recognizable DNA sequence longer. As a result, a DNA-binding protein acquires a wider range for interaction with DNA, which increases the affinity to DNA and, at the same time, gives higher selectivity of nucleotide sequences. In some of homeodomains, however, not only HTH motifs but also N-terminal or C-terminal arm is involved in the recognition of bases. Therefore, the recognition range is about 7 to 10 base pairs and the area of proteins that can interact with DNA becomes wider. Thus, a homoedomain is capable of binding to DNA with only one domain. hTRF1 and hTRF2 bind to DNA by dimer formation. There are no interactions between DNA-binding proteins; one DNA-binding domain is capable of binding to DNA by itself. This is enabled by the N-terminal arm which recognizes the minor groove of DNA, as seen in homeodomains. In homeodomains whose structures are determined as complexes with DNA, Arg recognizes AT-rich sequences in minor groove of DNA in most cases. Although Lys recognizes AT-rich sequence in MATa1, it is not binding performed by one DNA-binding motif alone because MATa1 and MATα2 form heterodimers with DNA and the recognition helices bind to the major groove of DNA from the same side to the opposite side in such a manner that the helices are oriented to the same direction. When Arg, not Lys, recognizes AT-rich sequences, adjacent bases and phosphate groups are also recognized simultaneously. Thus, Arg can recognize DNA with higher affinity. Even in homeodomains whose structures are not determined as complexes with DNA, Arg of the fifth residue is almost conserved. Thus, it is believed that Arg is used preferentially to Lys in the recognition of DNA in the minor groove. These results of NMR and SPR analyses suggest that hTRF2 has a lower affinity to DNA than hTRF1 because hTRF2 recognizes DNA in the minor groove with Lys. Besides, hTRF2 has a smaller number of hydrogen bonds in interactions at Ala471 and Ala484, hTRF2 dissociates from DNA more easily than hTRF1.

### Biological Implication

Both hTRF1 and hTRF2 have a C-terminal Myb domain and they bind to DNA by forming a homodimers. However, the two Myb domains do not bind to DNA cooperatively, but one Myb domain binds to DNA independently. The homology in the Myb domains of hTRF1 and hTRF2 is extremely high, and the results of structure analysis performed this time revealed that DNA recognition modes of hTRF1 and hTRF2 are almost identical. However, it is reported that the two Myb domains of hTRF1 homodimer bind not only to adjacent telomeric sequences but also to telomeric sequences located far apart to thereby bend *DNA in vitro.* hTRF2 does not have such a binding mode. It is reported that, as long as there is one TTAGGG sequence at the beginning of 3' overhang, hTRF2 is apt to bind to the double strand side located at the single-strand/double-strand telomeric junction. It is also reported that hTRF2 binds to D-loop within the t-loop structure. As a result of analyses made this time, it is supposed that, like the Myb domain of hTRF1, the Myb domain of hTRF2 is capable of binding to double-stranded telomeric sequences occupying the most part other than the region adjacent to 3' overhang. However, the binding site of hTRF2 has partiality to the 3' overhang. It is believed that hTRF2 once bound to double-stranded telomeric sequences are gathered to a region adjacent to the 3' overhang by the influence of other proteins interacting with hTRF2 (e.g., hRapl or Mrell complex) or single-stranded DNA, and then functions in t-loop formation or the like. It seems that binding to DNA with lower affinity than hTRF1 is advantageous for hTRF2 to move between telomeric sequences.

### "Experimental_Procedures"

### Preparation of Proteins and DNAs

DNA encoding TRF2-DBD was amplified by PCR using a plasmid encoding the full length of TRF2 with DNA primers (5' and 3') consisting of 40 bases. The amplified DNA was digested with restriction enzymes NdeI and EcoRI and ligated into pET23b vector, which was then transformed into *Escherichia coli* BL21 (DE3).

In addition to the above two primers, 5' primers and 3' primers encoding the amino acid sequences of the mutant sites were also prepared. For each mutant site, 5' side and 3' side were amplified by separate PCR. The resultant two DNAs and the 5' and 3' primers for TRF2-DBD were mixed and subjected to PCR, to thereby obtain a DNA encoding a mutant TRF2-DBD. The resultant DNA was treated with restriction enzymes and ligated in the same manner as described above for the DNA encoding TRF2-DBD, to thereby obtain an expression vector. The resultant vector was transformed into BL21 (DE3).

The primer sequences used for preparing mutants are described below. The upper row shows the 5' primer and the lower row shows the 3' primer. For K447R, only 5' primer is shown. For R496K, only the 3' primer is shown.
TRF2DBD
5'-ggtctcgcatatggaagacagtacaaccaatataac-3' (SEQ ID NO: 22)
5'-gcgggaattctcagttcatgccaagtcttttc-3' (SEQ ID NO: 23)
TRF2DBD (A471 S)
5'-ggaaactggtctgccatttctaaaaat-3' (SEQ ID NO: 24)
5'-agaaatggcagaccagtttccttcccc-3' (SEQ ID NO: 25)
TRF2DBD (A483S)
5'-aaccgaacatctgtgatgattaaggat-3' (SEQ ID NO: 26)
5'-aatcatcacagatgttcggttaacaaa-3' (SEQ ID NO: 27)
TRF2DBD (K447R)
5'-ggtctcgcatatggaagacagtacaaccaatataacaaaaaggcagaagtgg-3' (SEQ ID NO: 28)
TRF2DBD (R496K)
5'-ggaattctcagttcatgccaagttttttcatggtccg-5' (SEQ ID NO: 29)

Human TRF2 DNA binding domain (hTRF2-DBD; consisting of amino acids from Glu438 to Asn500 with a methionine residue at the N-terminus) and its six mutants were overexpressed in E. *coli* strain BL21(DE3) (Novagen) with pET23b vector. The cells were cultured at 37°C, and when OD₆₀₀ reached 0.5-0.6, 1 mM isopropyl-1-thio-β-D-galactopyranoside (IPTG) was added to induce protein expression at 25°C. After an additional 3 hr culture, the cells were harvested and resuspended in a buffer (50 mM potassium phosphate buffer [pH 7.0], 5 mM EDTA, 100 mM NaCl). For isotope labeling, M9 minimal medium containing ¹⁵NH₄Cl (0.15%) and/or [¹³C]-glucose (0.2%) was used for culture. TFR2-DBD was purified by the following procedures. Briefly, the cells were lysed by sonication on ice and then centrifuged (39,000 g). The supernatant was loaded to a cellulose phosphate column (P11; Whatman) equilibrated with a buffer (50 mM potassium phosphate buffer [pH 7.0], 5 mM EDTA, 100 mM NaCl) and eluted gradually with 150 mM, 200 mM and 250 mM NaCl. Fractions containing the protein of interest were collected and concentrated with Centriprep (Amicon) or Vivaspin (Vivascience) provided with a 3-kDa cutoff membrane to make the volume 2 ml of less. This sample was applied to a gel filtration column (Superdex 30; Pharmacia) column equilibrated with a buffer (50 mM potassium phosphate buffer [pH 7.0], 5 mM EDTA, 300 mM KCl). The identification and purity of the sample were assessed by MALDI-TOF mass spectroscopy and electrophoresis.

The oligonucleotide fragment (5'-GTTAGGGTTAGGG-3') (SEQ ID NO: 17 was purchased from Bex Co., Ltd. (Japan). Both strands of double helix were mixed in a buffer (50 mM potassium phosphate buffer [pH 7.0], 1 mM EDTA, 150 mM KCl) at an equimolar ratio and annealed by cooling slowly from 95°C to room temperature. The resultant sample was applied to a gel filtration column (Superdex 30; Pharmacia) column equilibrated with a buffer (50 mM potassium phosphate buffer [pH 7.0], 5 mM EDTA, 300 mM KCl).

To avoid aggregation, protein and DNA were dissolved separately in a buffer (50 mM potassium phosphate [pH 7.0], 150 mM KCl), and the protein solution was gradually added to the DNA solution until an equimolar ratio was obtained. Thus, a complex was formed. The resultant sample was dialyzed with 5 mM potassium phosphate (pH 6.9) in 10% D₂O (v/v).

### NMR Spectroscopy

TRF2-DBD-DNA complexes (1.0-1.5 mM) in 5 mM potassium phosphate buffer (pH 6.9) with 10% (v/v) or 100% D₂O were used in NMR experiments. The NMR experiments were carried out at 303 K on Bruker DMX-600 and -800. Protein backbone resonance assignments were obtained from 3D HN(CO)CA, 3D HNCA, 3D HNCO (Grzesiek, S. & Bax, A. Improved three-dimensional triple resonance NMR techniques applied to a 31 kDa protein. J.Magn. Reson. 96, 432-440. (1992a)), 3DHN (CA) CO, 3D CBCANH and 3D CBCA(CO)NH (Grzesiek S and Bax A (1992b) Correlating backbone amide and side-chain resonances in larger proteins by multiple relayed triple resonance NMR. J. Am. Chem. Soc. 114, 6291-6293). Protein side chain resonance assignments were obtained from 3D HBHA(CO)NH(1992b), 3D HCCH-TOCSY (Kay LE, Xu G-Y, Singer AU, Muhandiram DR and Forman-Kay JD (1993) A gradient-enhanced HCCH-TOCSY experiment for recording side-chain 1H and 13C correlations in H2O sample of protein. J. Magn. Reson. B., 101, 333-337), 3D HCCH-COSY, 3D 15N-edited NOESY, and 3D 15N-edite TOCSY experiments. ³JHNα coupling constants for dihedral ϕ restraints in the main chain were measured by 3D HNHA (Vuister GW and Bax A (1993) Quantitative J correlation: a new approach for measuring homonuclear three bond J-(HN-Hα) coupling constants in 15N-enriched protein. J. Am. Chem. Soc., 115, 7772-7777) experiments.

DNA resonance assignments and intramolecular distance restraints were obtained from 2D NOESY, 2D TOCSY, and 2D DQF-COSY with a 13C or 13C/15N-filtered pulse scheme (Ogura K, Terasawa H and Inagaki F (1996) An improved double-tuned and isotope-filtered pulse scheme based on a pulse field gradient and a wide-band inversion shaped pulse. J. Biomol. NMR, 8, 492-498). Intermolecular distance restraints were obtained from a 3D 13C-edited (F1), 13C-filtered (F3) NOESY experiment and a 3D 15N-edited (F2), 15N/13C-filtered (F3) NOESY experiment (Ogura K, Terasawa H and Inagaki F (1996) An improved double-tuned and isotope-filtered pulse scheme based on a pulse field gradient and a wide-band inversion shaped pulse. J. Biomol. NMR, 8, 492-498). All NMR spectra were processed and analyzed using NMRPipe (Delaglio F, Grzesiek S, Vuister GW, Zhu G, Pfeifer J and Bax A (1995) NMRPipe: A multidimensional spectral processing system based on UNIX PIPES. J. Biomol. NMR, 6, 277-293) and PIPP (Garret DS, Powers R, Gronenborm AM and Clore GM (1991) A common sense approach to peak picking in two-, three-, and four-dimentinal spectra using automatic computer analysis of contour diagrams. J. Magn. Reson., 99, 214-220) softwares.

### Structure Calculations

Interproton distance constraints for TRF2-DBD were derived from the cross-peak intensities of the NOESY spectra. NOEs were classified into four distance ranges; 1.8-3.0, 2.3-4.0, 2.3-5.0, and 2.3-6.0 Å, corresponding to strong, medium, weak, and very weak NOEs, respectively. In addition, torsion angle restraints were derived from the ³JHNα coupling constants. The restraint angle ranges were -90°<ϕ< -40° for ³JHNα < 5.5Hz and -160° <ϕ<-80° for ³JHNα > 8.5Hz. The intra-DNA NOEs were classified into four distance ranges, 1.8-3.0, 2.3-4.0, 2.3-5.0, and 2.3-6.0Å, corresponding to strong, medium, weak, and very weak NOEs, respectively. Pseudo-atom correction was applied to the upper limit. Hydrogen bond restraints within the DNA were used to maintain the base pairs. Watson-Crick base pairing was maintained in the DNA by the following hydrogen bond restraints: for GC base pair, r_{G(N1)-C(N3)} = 2.95 ± 0.2 Å, r_{G(N2)-C(O2)} = 2.86 ± 0.2 Å, and r_{G(O6)-C(N4)} = 2.91 ± 0.2 Å and for TA base pair, r_{A(N6)-T(O4)} = 2.95 ± 0.2 Å, and r_{A(N1)-T(N3)}= 2.82 ± 0.2 Å (Grpnenborn, A.M. & Clore, G.M. Three-dimentional structure of proteins in by nuclear magnetic resonance spectroscopy. Protein Seq Date Anal. 2, 1-28. (1989)). Loose torsion angle restraints for the DNA were used, covering both A- and B-form DNA conformers (α = -65°± 50°, β = 180° ± 50°, γ = 60° ± 50°, ε = 180° ± 50°, and ζ = -85° ± 50°) (Omichinski, J.G., Pedone, P.V., Felsenfeld, G., Gronenborn, A.M. & Clore, G.M. The solution structure of a specific GAGA factor-DNA complex reveals a modular binding mode. Nat. Struct. Biol. 4, 122-132. (1997); Wojciak, J.M., Connolly, K.M. & Clubb, R.T. NMR structure of the Tn916 integrase-DNA complex. Nat. Struct. Biol. 6, 366-373. (1999)). These hydrogen bond and torsion angle restraints for the DNA are justified, because the pattern of NOEs for the DNA is typical of B-form DNA (Wuthrich, K. NMR of Proteins and Nucleic Acids. Wiley, New York. (1986)).

First, 200 structures of protein alone were calculated using crystallography and NMR System (CNS; Yale University). Secondly, the structures of hTRF2-DNA complexes were calculated using all NOEs with simulated annealing protocols, starting from the 200 structures of the calculated hTRF2-DBDs and B-form DNA. B-form DNA was placed 50 Å away from the protein in various orientations. In total, 200 structures of the hTRF2-DNA complex were calculated. Of these, 52 structures showed neither violation greater than 0.3 Å for the distance constraints nor 5° for the dihedral restraints. Finally, the 20 structures with the lowest energy and no structural contradiction were selected.

### Surface Plasmon Resonance Analyses

Affinity analysis was performed using a Biacore 3000 instrument. All experiments were performed at 293 K using a buffer containing 10 mM HEPES-KOH, 3 mM EDTA, 180 mM KCl and 0.003% Triton X-100 (v/v) (pH 6.8). Flow cells of an SA streptavidin sensor chip were coated with the 13mer oligonucleotide biotinylated. The proteins were injected into flow cells over 3-5 min at a flow rate of 10 µL/min until the reaction of protein with DNA had been equilibrated. Bound proteins were removed by a 30-sec wash with 2M KCl. An equilibrium dissociate constant (Kd) was calculated from a Scatchard analysis of the RU values in the equilibrium region of the sensorgram at each analyte concentration. The affinity data were analyzed using BIAevaluation ver. 3.2 software.

**Table 1**

| Structural statistics for 20 structures of hTRF2complex and 25 structures of hTRF2 | | | | |
|---|---|---|---|---|
| Protein | | | | |
| | Distance restraints | | | |
| | | itra residue (i-j=0) | 218 | 196 |
| | | medium range (\|i-j\|<5) | 573 | 511 |
| | | long range (\|i-j\|≧5) | 231 | 203 |
| | total | | 1022 | 910 |
| | Dihedral angle retraints φ | | 38 | 42 |
| DNA | | | | |
| | Distance restraints | | | |
| | | intra residue | 131 | |
| | | sequential | 162 | |
| | | interstrand | 4 | |
| | total | | 297 | |
| Protein-DNA | | | 93 | |
| TOTAL | | | 1450 | |
| | Statistic for structure calculations | | <SA> | <SA> |
| | R.m.s. deviations from experimental restraints | | | |
| | | NOE(Å) | (4.70±0.70) × 10⁻³ | (2.46±0.06) × 10⁻³ |
| | | dihedrals(deg.) | (1.93±1.18)×10⁻² | (5.28±3.49) × 10⁻² |
| | | | | |
| | R.m.s. deviations from ideal restraints | | | |
| | | bonds(Å) | (1.10±0.05)×10⁻³ | (1.29±0.03) ×10⁻³ |
| | | angles(deg.) | (2.85±0.04) × 10⁻¹ | (4.64±0.03) × 10⁻¹ |
| | | impropers(deg.) | (1.60±0.07) × 10⁻¹ | (3.39±0.04) × 10⁻¹ |
| | complex:residue 447-496 for protein, base-DNA 1-11(3'-13') for DNA | | | |
| | free:residue 450-496 for protein | | | |
| | R.m.s. deviations of Atomis coordinates (Å) | | backbone/all heavy atoms | backbone/all heavy atoms |
| | | Protein | 0.43±0.09/0.81±0.08 | 0.49±0.09/0.95±0.0.08 |
| | | DNA | 0.43±0.14/0.38±0.13 | |
| | | Protein-DNA | 0.51 ±0.11/0.68±0.09 | |
| | PROCHEK Ramachndran plotstastistcs (%) | | | |
| | | Residues in most favoured regions | 83.5 | 81.2 |
| | | Residues in additinal allowed regions | 15.5 | 16.1 |
| | | Residues in generously allowed regions | 0.9 | 2.7 |
| | | Residues in disallowed regions | 0.0 | 0.0 |

**Table2**

| 10mM HEPES-KOH pH6.8, 3mM EDTA, 180mM KCl, 0.003%(v/v) X-100 | | | | | | | | |
|---|---|---|---|---|---|---|---|---|
| | | | | | | | | KD (M) |
| | hTRF1 | hTRF2 | qm | dm , | K447R | A471S | A484S | R496K |
| tr13 | (1.86±0.06) × 10⁻⁷ | (7.48±0.21) × 10⁻⁷ | (1.9±0.09)× 10⁻⁷ | (3.85±0.17) × 10⁻⁷ | (2.97±0.18) × 10⁻⁷ | (4.82±0.20) × 10⁻⁷ | (5.50±0.17) × 10⁻⁷ | (7.64±0.21) × 10⁻⁷ |
| T3G | (7,95±0.41) × 10⁻⁷ | (5.94±0.24) × 10⁻⁶ | (9.02±0.39) ×10⁻⁶ | (2.58±0.14) × 10⁻⁶ | (2.19±0.13) × 10⁻⁶ | (3.07±0.13) × 10⁻⁶ | (4.03±0.15) × 10⁻⁶ | (4.92±0.17) × 10⁻⁶ |
| G7C | (1.22±0.22)×10⁻⁵ | (5.33±0.88) × 10⁻⁵ | (4.44±0.43) × 10⁻⁶ | (3.38±0.32) × 10⁻⁵ | (2.45±0.17) × 10⁻⁵ | (3.23±0.28) × 10⁻⁵ | (5.07±0.47) × 10⁻⁵ | (8.70±1.13) × 10⁻⁵ |
| T9G | (3.14±0.14) × 10⁻⁶ | (1.10±0.06) × 10⁻⁴ | (2.65±0.09) × 10⁻⁵ | (4.79±0.25) × 10⁻⁵ | (3.70±0.21) × 10⁻⁵ | (4.42±0.13) × 10⁻⁵ | (6.91±0.35) × 10⁻⁵ | (1.14±0.12) × 10⁻⁴ |

All publications, patents, and patent applications cited herein are incorporated herein by reference in their entity.

### Industrial Applicability

According to the present invention, a TRF2 mutant which has a much higher ability to bind to telomeric duplex DNA than wild-type TRF2 is provided. The mutant of the invention may be used for controlling events in which TRF2 is involved (e.g., cancer, senescence, apoptosis, etc.). For example, it is believed that senescence or apoptosis can be prevented by using the mutant of the invention.

Further, according to the present invention, the structure and function of complexes composed of telomeric protein TRF2 DNA-binding domain and telomeric duplex DNA have been analyzed. Based on the results, it has become possible to screen for drugs capable of regulating the DNA-binding ability of TRF2.

### SEQUENCE LISTING FREE TEXT

<SEQ ID NO: 1>
   SEQ ID NO: 1 shows the DNA sequence of wild-type hTRF2-DBD.
<SEQ ID NO: 2>
   SEQ ID NO: 2 shows the amino acid sequence of wild-type hTRF2-DBD.
<SEQ ID NO: 3>
   SEQ ID NO: 3 shows the DNA sequence of mutant K447R.
<SEQ ID NO: 4>
   SEQ ID NO: 4 shows the amino acid sequence of mutant K447R.
<SEQ ID NO: 5>
   SEQ ID NO: 5 shows the DNA sequence of mutant A471S.
<SEQ ID NO: 6>
   SEQ ID NO: 6 shows the amino acid sequence of mutant A471S.
<SEQ ID NO: 7>
   SEQ ID NO: 7 shows the DNA sequence of mutant A484S.
<SEQ ID NO: 8>
   SEQ ID NO: 8 shows the amino acid sequence of mutant A484S.
<SEQ ID NO: 9>
   SEQ ID NO: 9 shows the DNA sequence of mutant R496K.
<SEQ ID NO: 10>
   SEQ ID NO: 10 shows the amino acid sequence of mutant R496K.
<SEQ ID NO: 11>
   SEQ ID NO: 11 shows the DNA sequence of mutant qm.
<SEQ ID NO: 12>
   SEQ ID NO: 12 shows the amino acid sequence of mutant qm.
<SEQ ID NO: 13>
   SEQ ID NO: 13 shows the DNA sequence of mutant dm.
<SEQ ID NO: 14>
   SEQ ID NO: 14 shows the amino acid sequence of mutant dm.
<SEQ ID NO: 15>
   SEQ ID NO: 15 shows the DNA sequence of wild-type hTRF2.
<SEQ ID NO: 16>
   SEQ ID NO: 16 shows the amino acid sequence of wild-type hTRF2.
<SEQ ID NO: 17>
   SEQ ID NO: 17 shows the DNA sequence of one strand of duplex DNA tr13.
<SEQ ID NO: 18>
   SEQ ID NO: 18 shows the DNA sequence of the other strand of duplex DNA tr13.
<SEQ ID NO: 19>
   SEQ ID NO: 19 shows the DNA sequence of telomeric DNA mutant T3G.
<SEQ ID NO: 20>
   SEQ ID NO: 20 shows the DNA sequence of telomeric DNA mutant G7C.
<SEQ ID NO: 21>
   SEQ ID NO: 21 shows the DNA sequence of telomeric DNA mutant T9G.
<SEQ ID NO: 22>
   SEQ ID NO: 22 shows the sequence of 5' primer for amplifying TRF2-DBD.
<SEQ ID NO: 23>
   SEQ ID NO: 23 shows the sequence of 3' primer for TRF2-DBD.
<SEQ ID NO: 24>
   SEQ ID NO: 24 shows the sequence of 5' primer for TRF2-DBD mutant A471S.
<SEQ ID NO: 25>
   SEQ ID NO: 25 shows the sequence of 3' primer for TRF2-DBD mutant A471S.
<SEQ ID NO: 26>
   SEQ ID NO: 26 shows the sequence of 5' primer for TRF2-DBD mutant A483S.
<SEQ ID NO: 27>
   SEQ ID NO: 27 shows the sequence of 3' primer for TRF2-DBD mutant A483S.
<SEQ ID NO: 28>
   SEQ ID NO: 28 shows the sequence of 5' primer for TRF2-DBD mutant K447R.
<SEQ ID NO: 29>
   SEQ ID NO: 29 shows the sequence of 3' primer for TRF2-DBD mutant R496K.

## Claims

1. A TRF2 DNA-binding domain mutant protein comprising:
(a) a TRF2 DNA-binding domain mutant protein having an amino acid sequence as shown in SEQ ID NO: 2 but with at least one substitution selected from the group consisting of substitution of the lysine residue with arginine at position 10, substitution of the alanine residue with serine at position 34, substitution of the alanine residue with serine at position 47 and substitution of the arginine residue with lysine at position 59; or
(b) a TRF2 DNA-binding domain mutant protein having an amino acid sequence of the mutant protein of (a) above but with one or several amino acid residues other than the amino acid residues at positions 10, 34, 47 and 59 being deleted, substituted or added,. and which has a higher binding ability to a duplex DNA comprising a sequence represented by 5'-TTAGGG-3' than a wild-type TRF2 DNA-binding domain protein having an amino acid sequence as shown in SEQ ID NO: 2; or a salt of (a) or (b).

2. The TRF2 DNA-binding domain mutant protein or a salt thereof according to claim 1, wherein the mutant protein of (a) is any one of the following (ia) to (via):
(ia) a protein having an amino acid sequence as shown in SEQ ID NO: 2 but with the lysine residue at position 10 being substituted with arginine;
(iia) a protein having an amino acid sequence as shown in SEQ ID NO: 2 but with the alanine residue at position 34 being substituted with serine;
(iiia) a protein having an amino acid sequence as shown in SEQ ID NO: 2 but with the alanine residue at position 47 being substituted with serine;
(iva) a protein having an amino acid sequence as shown in SEQ ID NO: 2 but with the arginine residue at position 59 being substituted with lysine;
(va) a protein having an amino acid sequence as shown in SEQ ID NO: 2 but with the lysine residue at position 10 being substituted with arginine, the alanine residue at position 34 being substituted with serine, the alanine residue at position 47 being substituted with serine and the arginine residue at position 59 being substituted with lysine;
(via) a protein having an amino acid sequence as shown in SEQ ID NO: 2 but with the lysine residue at position 10 being substituted with arginine and the alanine residue at position 47 being substituted with serine;
(viia) a protein having an amino acid sequence as shown in SEQ ID NO: 2 but with the alanine residue at position 34 being substituted with serine and the alanine residue at position 47 being substituted with serine; or
(viiia) a protein having an amino acid sequence as shown in SEQ ID NO: 2 but with the lysine residue at position 10 being substituted with arginine, the alanine residue at position 34 being substituted with serine and the alanine residue at position 47 being substituted with serine.

3. An isolated DNA encoding the protein according to claim 1.

4. A recombinant vector comprising the DNA according to claim 3.

5. A transformant comprising the recombinant vector according to claim 4.

6. A method of producing a TRF2 DNA-binding domain mutant protein, comprising culturing a host transformed with the DNA according to claim 3 and recovering the TRF2 DNA-binding domain mutant protein from the resultant culture.

7. An antibody to the TRF2 DNA-binding domain mutant protein or a salt thereof according to claim 1.

8. A protein comprising the TRF2 DNA-binding domain mutant protein according to claim 1; or a salt thereof.

9. A complex of the protein according to claim 1 or 8 and a DNA.

10. A DNA having a nucleotide sequence as shown in SEQ ID NO: 17 but with at least one substitution selected from the group consisting of substitution of the T at position 3 with G, substitution of the G at position 7 to C and substitution of the T at position 9 to G.

11. The DNA according to claim 10, which is any one of the following (ib) to (iiib):
(ib) a DNA having a nucleotide sequence as shown in SEQ ID NO: 17 with the T at position 3 being substituted with G;
(iib) a DNA having a nucleotide sequence as shown in SEQ ID NO: 17 with the G at position 7 being substituted with C; or
(iiib) a DNA having a nucleotide sequence as shown in SEQ ID NO: 17 with the T at position 9 being substituted with G.

12. A method of screening for substances which are capable of regulating the binding of telomeric DNA to TRF2, comprising analyzing whether or not a TRF2 DNA-binding domain having an amino acid sequence as shown in SEQ ID NO: 2 or a protein comprising said domain interacts with a test substance at least at one amino acid site selected from the group consisting of the lysine residue at position 10, the alanine residue at position 34, the alanine residue at position 47 and the arginine residue at position 59, wherein said test substance is judged to be capable of regulating the binding of telomeric DNA to TRF2 when said test substance interacted with said domain or said protein.

13. The method according to claim 12, wherein whether or not a TRF2 DNA-binding domain having an amino acid sequence as shown in SEQ ID NO: 2 or a protein comprising said domain interacts with a test substance at least at one amino acid site selected from the group consisting of the lysine residue at position 10, the alanine residue at position 34, the alanine residue at position 47 and the arginine residue at position 59 is analyzed in the presence of a duplex DNA comprising a sequence represented by 5'-TTAGGG-3'.
